(19) **Europäisches Patentamt / European Patent Office / Office européen des brevets**

(11) **EP 2 512 479 B1**

(12) **EUROPEAN PATENT SPECIFICATION**

(45) Date of publication and mention
of the grant of the patent:
**30.03.2016 Bulletin 2016/13**

(51) Int Cl.:
*A61K 45/06* (2006.01)   *A61K 31/497* (2006.01)
*A61K 31/198* (2006.01)   *A61K 31/519* (2006.01)
*A61P 9/08* (2006.01)

(21) Application number: **10838297.9**

(22) Date of filing: **17.12.2010**

(86) International application number:
**PCT/US2010/061054**

(87) International publication number:
**WO 2011/075655 (23.06.2011 Gazette 2011/25)**

(54) **COMPOSITIONS FOR TREATING PERIPHERAL VASCULAR DISEASE**

ZUSAMMENSETZUNGEN ZUR BEHANDLUNG VON PERIPHERER GEFÄSSERKRANKUNG

COMPOSITIONS DESTINÉES AU TRAITEMENT DE MALADIES VASCULAIRES PÉRIPHÉRIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2009 US 287967 P**

(43) Date of publication of application:
**24.10.2012 Bulletin 2012/43**

(73) Proprietor: **Exodos Life Sciences Limited Partnership
Chapel Hill, NC 27517 (US)**

(72) Inventors:
- **FRANGAKIS, Crist, J.
Chapel Hill
NC 27516 (US)**
- **LEIGHTON, Harry, J.
Rockport
ME 04856 (US)**

(74) Representative: **Bösl, Raphael Konrad
Isenbruck Bösl Hörschler LLP
Patentanwälte
Prinzregentenstraße 68
81675 München (DE)**

(56) References cited:
WO-A1-2010/062366   US-A1- 2002 182 162
US-A1- 2004 110 843   US-A1- 2005 009 835
US-A1- 2009 053 283

- BAKST R ET AL: "Raynaud's phenomenon: Pathogenesis and management", JOURNAL OF THE AMERICAN ACADEMY OF DERMATOLOGY, C.V. MOSBY, ST. LOUIS, MO, US, vol. 59, no. 4, 1 October 2008 (2008-10-01), pages 633-653, XP025470534, ISSN: 0190-9622, DOI: 10.1016/J.JAAD.2008.06.004 [retrieved on 2008-07-24]
- FRIES ET AL.: 'Sildenafil in the Treatment of Raynaud?s Phenomenon Resistant to Vasodilatory Therapy' CIRCULATION vol. 112, 2005, pages 2980 - 2985, XP008155105
- GRESSER ET AL.: 'ERECTILE DYSFUNCTION: COMPARISON OF EFFICACY AND SIDE EFFECTS OF THE PDE-5 INHIBITORS SILDENAFIL, VARDENAFIL AND TADALAFIL REVIEW OF THE LITERATURE' EUROPEAN JOURNAL OF MEDICAL RESEARCH vol. 7, 2002, pages 435 - 446, XP008155106

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND OF THE INVENTION**

This invention pertains to compositions as defined in the claims for use in methods for treating peripheral vascular disease, including Raynaud's phenomenon or syndrome. More particularly, the invention features compositions that contain one or more cyclic nucleotide phosphodiesterase inhibitors, which is sildenafil, derivatives, or salts thereof, in combination with one or more nitric oxide (NO) donors, which is L-arginine. These compositions may be applied topically to the extremities of a patient suffering from a peripheral vascular disease in order to improve vascular blood flow in affected areas, thereby alleviating one or more symptoms associated with the disease.

Raynaud's phenomenon is a medical condition characterized by a transient, reversible vasospasm of the peripheral arteries, affecting the patient's fingers, toes, ears, and/or nose. It occurs in about 3-5% of the population and can run in families. In susceptible individuals, it is usually triggered by exposure to cold or stress, followed by a color change of the affected body part, turning first white due to ischemia, then cyanotic and finally red due to reperfusion (erythrema). In addition, it can cause pain and sometimes paresthesia, and in rare instances, ulceration of the fingers and/or toes (and in some cases of the nose and/or ears).

Primary or idiopathic Raynaud's (Raynaud's disease) occurs without an underlying disease. Secondary Raynaud's (Raynaud's syndrome) occurs in association with an underlying disease - usually connective tissue disorders, such as scleroderma, systemic lupus erythematosus (SLE), Sjögren's syndrome, rheumatoid arthritis, and polymyositis, among others. Secondary Raynaud's may be associated with vasculitis, severe peripheral vascular disease, including Buerger's disease, and rarely malignancy or chemotherapy. Vibration trauma, such as in jackhammer operators, may precipitate Raynaud's, though there is debate in the literature as to whether this should be considered primary or secondary.

Primary Raynaud's phenomenon is more common in women than men and is characterized by the presence of vasospasm alone, which is not associated with any serious disorder. It may cause superficial ulceration of digital tips, but gangrene rarely occurs. In contrast, secondary Raynaud's phenomenon may result in significant morbidity, presenting with digital ulcers and life-threatening consequences. Digital ulcers in secondary Raynaud's are usually very painful, limit hand function and can lead to soft tissue infections or, in severe cases, to gangrene requiring digital amputation. Examination of nail fold capillaries is used to confirm Raynaud's phenomenon associated with systemic sclerosis.

Vasospasm is a normal reaction to cold or temperature change; however, it is exaggerated in primary Raynaud's with increased vasospasm. Many factors are upregulated in patients with primary Raynaud's, and more so in patients with secondary Raynaud's, including calcitonin gene-related product (CGRP), serotonin receptors, and endothelin. Genetics can be associated with Raynaud's, but it appears to be polygenic and differs across nationalities. For example, if someone has primary Raynaud's they may have a family history of primary or secondary Raynaud's. Similarly, someone with secondary Raynaud's may have a family history of connective tissue disease with or without Raynaud's or even a family history of primary Raynaud's.

To date, there is no effective treatment for primary Raynaud's disease. Self-help measures such as controlling stress, avoiding exposure to cold sources or warming up digits during a vasospastic episode are generally recommended. Drugs are of limited use for the primary form, while vasodilators, primarily calcium channel blockers, are often prescribed for secondary Raynaud's phenomenon.

The molecular mechanisms behind Raynaud's phenomenon are not yet well understood and will probably be different for the primary and secondary forms of the disease. Independent of etiology, manifestations of Raynaud's phenomenon encompass vasospasm of digital arteries and arterioles revealing impaired vasomotor control. Regulation of vascular tone depends upon the interaction among endothelium, smooth muscle and the autonomic nervous system that innervates blood vessels. Defective control of vascular tone may be associated with causes intrinsic to the vessel wall, such as abnormalities of structural or functional origin, or may be due to extrinsic factors stemming from impaired neural regulation or intravascular circulating factors, such as platelet activation or fibrinolysis.

Endothelial cells secrete vasoconstricting substances (e.g., endothelin-1 [ET-1]) or vasodilating substances (e.g., nitric oxide [NO], prostacyclin) that participate in the regulation of vascular tone. Thus, endothelial dysfunction caused by different conditions may lead to imbalance in the secretion of vasoactive mediators, shifting the equilibrium towards excessive vasoconstriction, which leads to vasospasm. For instance, the elevated levels of the vasoconstrictor ET-1 found in patients with systemic sclerosis suggest that endothelium-dependent factors may be important in Raynaud's phenomenon. Abnormal regulation of vasodilating substances, such as NO, has also been reported. A recent study showed reduced nitro-tyrosine levels in patients with primary Raynaud's phenomenon compared to patients with systemic sclerosis or healthy controls, suggesting potentially up-regulated degradation of nitrated proteins. Nitrated protein residues are indicative of the formation of reactive oxygen species (ROS). Given the milder nature of primary Raynaud's symptoms, lower nitro-tyrosine levels were suggested to be protective. Also, increased NO levels have been observed in patients with Raynaud's phenomenon and limited systemic sclerosis. However, patients with diffuse systemic sclerosis (more severe) presented with normal NO levels, but increased nitrated protein expression and circulating asymmetric dimethylarginine, an endogenous inhibitor of endothelial nitric oxide synthase (NOS). These mixed findings reflect the complex role of NO metabolism in the pathophysiology of Raynaud's phenomenon.

NO is synthesized, at least in part, from L-arginine by a family of enzymes known as nitric oxide synthases (NOS). It is believed that NOS convert L-arginine, NADPH, and oxygen into citrulline, NADH, and NO. NOS occur in several isoforms: an endothelial nitric oxide synthase (eNOS), a macrophage or inducible nitric oxide synthase (iNOS), and a neuronal nitric oxide synthase (nNOS). Despite its name, eNOS has been detected not only in endothelial cells and blood vessels, but also in epithelium of tissues including, but not limited to, bronchial cells and neurons of the brain, especially in the pyramidal cells of the hippocampus. Furthermore, iNOS has been detected not only in macrophages, but also in cells such as hepatocytes, chrondrocytes, endothelial cells and fibroblasts, in particular under conditions of endothelial damage or as part of a response to injury.

Recent studies suggest that NOS inhibitors may be associated with endothelial vasodilator dysfunction, as with Raynaud's. In particular, asymmetric dimethylarginine (ADMA), and to a lesser extent N-monomethylarginine (NMA), are associated with endothelia vasodilator dysfunction. Patients with coronary and peripheral arterial disease and those with renal failure have greater amounts of plasma ADMA. However, it has been shown that while exogenous ADMA vasoconstricts vascular rings in vitro, the vasoconstriction effect can be reversed by L-arginine.

Formation of NO by eNOS is thought to play an important role in normal blood pressure regulation and endothelial dysfunction. eNOS is the predominant synthase in the endothelium and is active under basal conditions. Since intracellular levels of L-arginine are normally greater that NOS enzyme, NO synthesis generally does not depend on extracellular supplementation. Under certain circumstances, as with Raynaud's, local L-arginine concentrations might become rate limiting.

As a free radical gas, NO has an extremely short half-life. In certain instances, it may be desirable to increase the effective amount of NO in a cell, tissue, or organ in order to induce vascular relaxation, vascular dilation, vascularization, oxygenation, or other NO mediated biological process.

Systemic sclerosis features impaired vasodilatation since damaged endothelium compromises NO production. This endothelium-dependent vasodilatation plays an important role in disease pathogenesis. Since nitroglycerin is an NO donor with an endothelium-dependent vasodilating effect, it has been proposed as a treatment option to ameliorate the symptoms of systemic sclerosis, including Raynaud's phenomenon. Topical forms of nitroglycerin are have been used to avoid associated side effects, including headache and hypotension.

For example, MediQuest Therapeutics evaluated the safety and efficacy of a topical organogel containing nitroglycerin (MQX-503) in a randomized, double-blind, placebo-controlled phase III clinical trial. Thirty-six patients were included in this study to determine the response to two doses of the topical nitroglycerin formulation. Patients presented with moderate to severe Raynaud's phenomenon, primary or secondary to scleroderma or other autoimmune diseases, and were exposed to a controlled cold challenge. Within 5 min of local nitroglycerin application in the fingers, enhanced blood flow was observed. Secondary outcomes include a reduction in the onset of digital ulcers in patients with scleroderma. MediQuest has scheduled a second, larger-scale study to examine the efficacy of this topical nitroglycerin formulation in reducing the number of vasospasm attacks, improving Raynaud's assessment score and decreasing associated symptoms, as well as safety.

A nitroglycerin tape formulation (MILLISROL®) was also evaluated by Japanese researchers in a pilot study of 25 patients with systemic sclerosis. Finger temperature, determined by thermography, was increased in 60% of scleroderma patients after nitroglycerin tape application, while the temperature in the placebo tape group remained unaffected. This finding suggested improved peripheral circulation after topical nitroglycerin treatment.

The vasodilating effects of NO are mediated by the cyclic nucleotide cyclic guanosine monophosphate (cGMP). Phosphodiesterase type 5 (PDE5) inhibitors prevent cGMP degradation, thereby increasing its accumulation in vascular smooth muscle cells. This therapeutic strategy has proved successful in the treatment of erectile dysfunction and pulmonary hypertension. The efficacy of PDE5 inhibitors in Raynaud's phenomenon has been investigated in several clinical trials that reported overall improvement in vasospastic symptoms and a favorable safety profile. In particular, orally administered sildenafil exhibited efficacy in a double-blind, placebo-controlled, crossover study conducted in 18 patients with secondary Raynaud's phenomenon who showed resistance to previous vasodilator therapy. Oral administration of 50 mg sildenafil twice daily for 4 weeks produced a reduction in the frequency and duration of Raynaud's attacks compared to placebo. Two patients suffering from primary Raynaud's showed similar improvement. In all patients who had digital ulcers at baseline, ulcer healing was observed after sildenafil treatment, with total ulcer remission in 2 cases. Interestingly, improvement in Raynaud's symptoms correlated with a more than 4-fold increase in mean capillary flow velocity in sildenafil-treated patients. Another randomized, double-blind, crossover study in 15 patients with Raynaud's phenomenon (unspecified whether primary or secondary) reported a 75% increase in forearm blood flow after sildenafil treatment. The study compared oral sildenafil to the antioxidant $\alpha$-tocopherol, which demonstrated no effects on blood flow parameters. Results from a small retrospective study of patients with Raynaud's phenomenon secondary to scleroderma that failed conventional vasodilator treatment and were offered sildenafil were also encouraging: 80% of patients experienced a reduction in pain and frequency of Raynaud's phenomenon attacks, and digital ulcer healing was seen in 75% of patients who had digital ulcers at baseline. However, sildenafil failed to improve primary Raynaud's symptoms in another randomized, double-blind, crossover study.

Other PDE5 inhibitors, such as tadalafil, also reduced Raynaud's symptoms according to a small open-label pilot study in patients with scleroderma and lupus. Another study of oral administration of tadalafil 20 mg 2 or 3 times per week during 4 weeks showed a significant reduction in vasospastic attack frequency and duration and increased peripheral blood flow at the end of the treatment period compared to pentoxifylline 600 mg b.i.d. Interestingly, improvement was also seen after long-term treatment with tadalafil, indicating that acute vasodilatation may be only partially responsible for its effects. Also, tadalafil appeared to be a good alternative in a patient suffering from Raynaud's phenomenon secondary to chemotherapy for oral squamous cell carcinoma that did not respond to increasing doses of sildenafil. In this isolated case report, equipotent doses of tadalafil improved Raynaud's symptoms and increased capillary blood flow. Improved efficacy was attributed to the longer half-life of tadalafil (17.5 h vs. 3.8 h for sildenafil).

Finally, vardenafil was tested in an open-label pilot study carried out in 40 patients presenting with primary (18%) or secondary (82%) Raynaud's phenomenon. In this study with oral administration of vardenafil, the duration, number and severity of Raynaud's attacks were reduced by 60%, 50% and 53%, respectively, in vardenafil-treated patients. Improved clinical symptoms correlated with increased digital blood flow in 70% of the patients.

In summary, cGMP phosphodiesterase (PDE) inhibitors have been shown when administered orally to increase peripheral blood flow. These agents have also been reported to have therapeutic potential in peripheral vascular disease, such as Raynaud's; however their systemic vascular effects and adverse side effects (e.g., a decrease in blood pressure) have precluded their use as vasodilators. Likewise, NO producing compounds have been investigated in several clinical studies for the treatment of peripheral vascular disease with mixed results. For example, previous studies described the topical application of a nitric oxide precursor (arginine) to increase vascular blood flow. However, these agents failed to demonstrate sustained pharmacological activity due to its short duration of action, as cGMP, the intracellular messenger responsible for the biological activity, is quickly degraded by specific phosphodiesterase enzymes. Therefore, there is a need for safe and effective methods and compositions to treat peripheral vascular disease, as well as conditions associated with peripheral vascular disease.

## SUMMARY OF THE INVENTION

In a first aspect, the invention features a composition as defined in the claims for use in a method of treating a peripheral vascular disease the method including administering to a subject an effective amount of at least one phosphodiesterase type 5 inhibitor and at least one nitric oxide donor. In certain embodiments, the peripheral vascular disease is primary Raynaud's phenomenon, secondary Raynaud's phenomenon (e.g., where the condition associated with secondary Raynaud's phenomenon is one or more of systemic sclerosis, CREST syndrome, systemic lupus erythematosus, rheumatoid disease, rheumatoid arthritis, Sjögren's syndrome, or polymyositis). In other embodiments, the peripheral vascular disease is one or more of peripheral neuropathy, autonomic neuropathy, diabetic neuropathy, vasculitis, skin aging, necrotizing fasciitis, decubitus ulcers, anal fissure, diffused cutaneous systemic sclerosis, or frostbite.

In one embodiment of the first aspect, the method includes topical administration to the affected area of the subject. In another embodiment of the first aspect, the method includes oral administration.

In certain embodiments of the first aspect, the phosphodiesterase type 5 inhibitor and the nitric oxide donor are administered together in a pharmaceutical composition. In other embodiments of the first aspect, the phosphodiesterase type 5 inhibitor is administered orally and the nitric oxide donor is administered topically.

In yet another embodiment of the first aspect, the amount of the phosphodiesterase type 5 inhibitor is 1 mg to 500 mg daily (e.g., 1, 5, 10, 25, 50, 100, 250, and 500 mg) and the amount of the nitric oxide donor is 1 mg to 500 mg daily (e.g., 1, 5, 10, 25, 50, 100, 250, and 500 mg).

The phosphodiesterase type 5 inhibitor is selected from the group consisting of sildenafil, and derivatives and salts thereof (e.g., the citrate salt of sildenafil).

In another embodiment of the first aspect, the phosphodiesterase type 5 inhibitor has an IC50 of less than 100 nanomolar. In yet another embodiment of the first aspect, the phosphodiesterase type 5 inhibitor has a selectivity ratio in excess of 1000.

The nitric oxide donor is selected from arginine (e.g., L-arginine), and derivatives and salts thereof.

In a further embodiment of the first aspect, the invention features a composition for use in a method further including administering one or more of an a 5-HT$_{2B}$ antagonist, an $\alpha$-adrenergic receptor antagonist, a $\beta$-adrenergic receptor antagonist, an angiotensin receptor antagonist, an angiotensin converting enzyme inhibitor, an anticoagulant, an antidepressant, an antidiabetic agent, an antithrombotic, a calcium channel blocker, a cholesterol-lowering drug, a non-steroidal anti-inflammatory agent, a prostaglandin, a renin antagonist, a steroidal anti-inflammatory agent, or a thromboxane A2 agonist.

In a second aspect, the disclosure features a composition formulated for topical administration including at least one phosphodiesterase type 5 inhibitor (e.g., sildenafil, vardenafil, tadalafil, udenafil, lodenafil, gisadenafil, avanafil, gisadenafil, mirodenafil, parogrelil, SLx-2101, 3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one, 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine, 5-(2-ethoxy-5-morpholinoacetylphenyl)-1-methyl-3-n-propyl-1,6-dihydro-7H-pyrazolo[4,3-d]pyrimidin-7-one, 3-ethyl-5-[5-(4-ethylpiperazin-1-ylsulphonyl)-2-n-propoxyphenyl]-2-(pyridin-2-yl)methyl-2, 6-dihydro- 7H-pyrazolo[ 4,3-d]pyrimidin-7 -one, 3-ethyl-5-[5-(4-ethylpiperazin-1-ylsulphonyl)-2-(2-methoxyethoxy) pyridin-3-yl]-2(pyridin-2-yl)methyl-2,6-dihydro-7H-pyrazolo[4,3-d]pyrimidin-7-one, (+)-3-ethyl-5-[5-(4-ethylpiperazin-1-ylsulphonyl)-2-(2-methoxy-l(R)-methylethoxy)pyridin-3-yl]-2-methyl-2,6-dihydro-7H-pyrazolo[4,3-d]pyrimidin-7-one, 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, 5-[2-iso-butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one, 5-[2-ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one, 5-(S-acetyl-2-propoxy-3-pyridinyl)-3-ethyl-2-(1-isopropyl-3-azetidinyl)-2,6-dihydro-7H-pyrazolo[4,3-d]pyrimidin-7-one, 5-(5-acetyl-2-butoxy-3-pyridinyl)-3-ethyl-2-(1-ethyl-3-azetidinyl)-2,6-dihydro-7H-pyrazolo[4,3-d]pyrimidin-7-one, and derivatives and salts thereof), at least one nitric oxide donor (e.g., an organic nitrate ester, an organic nitrite ester, a S-nitrosylated compound, a diazenium diolate, a vasodilator, a citrulline, an arginine (e.g., L-arginine), and derivatives and salts thereof), and a pharmaceutically acceptable carrier.

In one embodiment of the second aspect, the composition includes from 1 mg to 500 mg (e.g., 1, 5, 10, 25, 50, 100, 250, and 500 mg) of one or more phosphodiesterase type 5 inhibitors and from 1 mg to 500 mg (e.g., 1,5, 10, 25, 50, 100, 250, and 500 mg) of one or more nitric oxide donors. In another embodiment, the composition includes from 10 mg to 100 mg of the phosphodiesterase type 5 inhibitor and from 10 mg to 100 mg of the nitric oxide donor. In yet another embodiment, the composition includes from 0.5% to 12.5% of the phosphodiesterase type 5 inhibitor and from 0.5% to 12.5% of the nitric oxide donor.

In a particular embodiment of the second aspect, the composition is formulated as a cream, a gel, a lotion, an ointment, a shampoo, a solution, a suspension, or a transdermal patch.

In a further embodiment of the second aspect, the composition further includes a permeation enhancer agent (e.g., a polyacrylic acid polymer, a polysaccharide gum, isopropyl myristate, isopropyl palmitate, dimethyl sulfoxide, decyl methyl sulfoxide, dimethylalanine amide of a medium chain fatty acid, dodecyl 2-(N,N-dimethylamino) propionate, tetradecyl (N,N-dimethylamino) acetate, dodecyl (N,N-dimethylamino) acetate, decyl (N,N-dimethylamino) acetate, octyl (N,N-dimethylamino) acetate, and dodecyl (N,N-diethylamino) acetate, or salts thereof).

In one embodiment of the second aspect, the composition is formulated as a solution and the solution further includes one of more of an antioxidant, an antimicrobial agent, a buffer, an emulsifying agent, a lipophilic solvent, a lubricating agent, a permeation enhancer agent, a stabilizer, a suspending agent, a tonicity adjusting agent, a viscosity increasing agent, or a wetting agent.

In a further embodiment of the second aspect, the composition further includes one or more of a 5-HT$_{2B}$ antagonist, an $\alpha$-adrenergic receptor antagonist, a $\beta$-adrenergic receptor antagonist, an angiotensin receptor antagonist, an angiotensin converting enzyme inhibitor, an anticoagulant, an antidepressant, an antidiabetic agent, an antithrombotic, a calcium channel blocker, a cholesterol-lowering drug, a non-steroidal anti-inflammatory agent, a prostaglandin, a renin antagonist, a steroidal anti-inflammatory agent, or a thromboxane A2 agonist.

In a third aspect, the disclosure features a composition formulated for oral administration including at least one phosphodiesterase type 5 inhibitor (e.g., sildenafil, vardenafil, tadalafil, udenafil, lodenafil, gisadenafil, avanafil, gisadenafil, mirodenafil, parogrelil, SLx-2101, 3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one, 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine, 5-(2-ethoxy-5-morpholinoacetylphenyl)-1-methyl-3-n-propyl-1,6-dihydro-7H-pyrazolo[4,3-d]pyrimidin-7-one, 3-ethyl-5-[5-(4-ethylpiperazin-1-ylsulphonyl)-2-n-propoxyphenyl]-2-(pyridin-2-yl)methyl-2, 6-dihydro- 7H-pyrazolo[4,3-d]pyrimidin-7 -one, 3-ethyl-5-[5-(4-ethylpiperazin-1-ylsulphonyl)-2-(2-methoxyethoxy)pyridin-3-yl]-2(pyridin-2-yl)methyl-2,6-dihydro-7H-pyrazolo[4,3-d]pyrimidin-7-one, (+)-3-ethyl-5-[5-(4-ethylpiperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one, 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, 5-[2-iso-butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one, 5-[2-ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one, 5-(S-acetyl-2-propoxy-3-pyridinyl)-3-ethyl-2-(1-isopropyl-3-azetidinyl)-2,6-dihydro-7H-pyrazolo[4,3-d]pyrimidin-7-one, 5-(5-acetyl-2-butoxy-3-pyridinyl)-3-ethyl-2-(1-ethyl-3-azetidinyl)-2,6-dihydro-7H-pyrazolo[4,3-d]pyrimidin-7-one, and derivatives and salts thereof), at least one nitric oxide donor (e.g., an organic nitrate ester, an organic nitrite ester, a S-nitrosylated compound, a diazenium diolate, a vasodilator, a citrulline, an arginine (e.g., L-arginine), and derivatives and salts thereof), and a pharmaceutically acceptable carrier.

In one embodiment of the third aspect, the composition includes from 1 mg to 500 mg (e.g., 1, 5, 10, 25, 50, 100, 250, and 500 mg) of the phosphodiesterase type 5 inhibitor and from 1 mg to 500 mg (e.g., 1,5, 10, 25, 50, 100, 250, and 500 mg) of the nitric oxide donor. In another embodiment, the composition includes from 10 mg to 100 mg of the phosphodiesterase type 5 inhibitor and from 10 mg to 100 mg of the nitric oxide donor. In yet another embodiment, the composition includes from 0.5% to 12.5% (w/w) of the phosphodiesterase type 5 inhibitor and from 0.5% to 12.5% (w/w) of the nitric oxide donor.

In a particular embodiment of the third aspect, the composition is formulated as a capsule, a pill, or a tablet.

In a further embodiment of the third aspect, the composition further includes one or more of a 5-HT$_{2B}$ antagonist, an α-adrenergic receptor antagonist, a β-adrenergic receptor antagonist, an angiotensin receptor antagonist, an angiotensin converting enzyme inhibitor, an anticoagulant, an antidepressant, an antidiabetic agent, an antithrombotic, a calcium channel blocker, a cholesterol-lowering drug, a non-steroidal anti-inflammatory agent, a prostaglandin, a renin antagonist, a steroidal anti-inflammatory agent, or a thromboxane A2 agonist.

In a fourth aspect, the disclosure features a kit including a composition formulated for topical administration including at least one phosphodiesterase type 5 inhibitor, at least one nitric oxide donor, and a pharmaceutically acceptable carrier; and instructions for administering the composition to a subject.

In a fifth aspect, the disclosure features a kit including a composition formulated for oral administration including at least one phosphodiesterase type 5 inhibitor, at least one nitric oxide donor, and a pharmaceutically acceptable carrier; and instructions for administering the composition to a subject.

*Definitions*

The term "administration" or "administering" refers to a method of giving a dosage of a pharmaceutical composition to a subject. The preferred method of administration may depend on a variety of factors, e.g., the components of the pharmaceutical composition or condition, and severity of the disease, disorder, or condition.

By administered "together" is meant that two or more compounds are formulated together in a single pharmaceutical composition that is administered to the subject.

By "affected area" is meant the region of the subject's extremities that display one or more symptoms of a peripheral vascular disease.

As used herein, the phrases "an effective amount" or "an amount effective to treat" when used in reference to treating a peripheral vascular disease or a condition associated with the peripheral vascular disease refers to an amount of one or more compounds that improves vasomotor control in a subject, relieves one or more symptoms caused by the peripheral vascular disease, or diminishes one or more symptoms caused by a condition associated with the peripheral vascular disease.

By "pharmaceutically acceptable" is meant that the compositions or components thereof are suitable for use in contact with a subject's tissue or bodily fluid without undue toxicity, incompatibility, instability, allergic response, and the like.

By "NO donor" or "nitric oxide donor" is meant a compound that increases the metabolic or enzymatic production of NO by NOS, that becomes converted into NO within a subject, or that contains, releases or donates a nitric oxide moiety.

By "NO moiety" is meant a functional group comprising one or more of -NO,-NO$_2$, or -NO$_3$.

By "NOS" is meant any isoform of nitric oxide synthase.

By "PDE5 inhibitor" is meant a compound that is a selective or nonselective inhibitor of the cGMP PDE5 isoenzyme.

By "subject" is meant a mammal, including, but not limited to, a human or non-human mammal.

As used herein, and as well understood in the art, "treatment" is an approach for obtaining beneficial or desired results, such as clinical results. Beneficial or desired results can include, but are not limited to, alleviation, amelioration, or prevention of a disease, a disorder, a condition, or one or more symptoms associated with a disease, a disorder, or a condition; diminishment of extent of disease, disorder, or condition; stabilized (i.e., not worsening) state of disease, disorder, or condition; delay or slowing the progress of the disease, disorder, or condition; and amelioration or palliation of the disease, disorder, or condition. "Treatment" can also mean prolonging survival as compared to expected survival if not receiving treatment. By "prevention" is meant that a prophylactic treatment is given to a subject who has or will have a disease, a disorder, a condition, or one or more symptoms associated with a disease, a disorder, or a condition. By "palliation" of a disease, a disorder, or a condition is meant that the extent and/or undesirable clinical manifestations of the disease, disorder, or condition are lessened and/or time course of the progression is slowed or lengthened, as compared to the extent or time course in the absence of treatment.

The recitation herein of numerical ranges by endpoints includes all numbers subsumed within that range (e.g. 1 to 5 includes 1, 1.5, 2, 2.75, 3, 3.80, 4, and 5).

As used herein, "a" or "an" means "at least one" or "one or more" unless otherwise indicated. In addition, the singular forms "a", "an", and "the" include plural referents unless the content clearly dictates otherwise. Thus, for example, reference to a composition containing "a compound" includes a mixture of two or more compounds.

Other features and advantages of the invention will be apparent from the following Detailed Description and from the claims.

**BRIEF DESCRIPTION OF THE DRAWINGS**

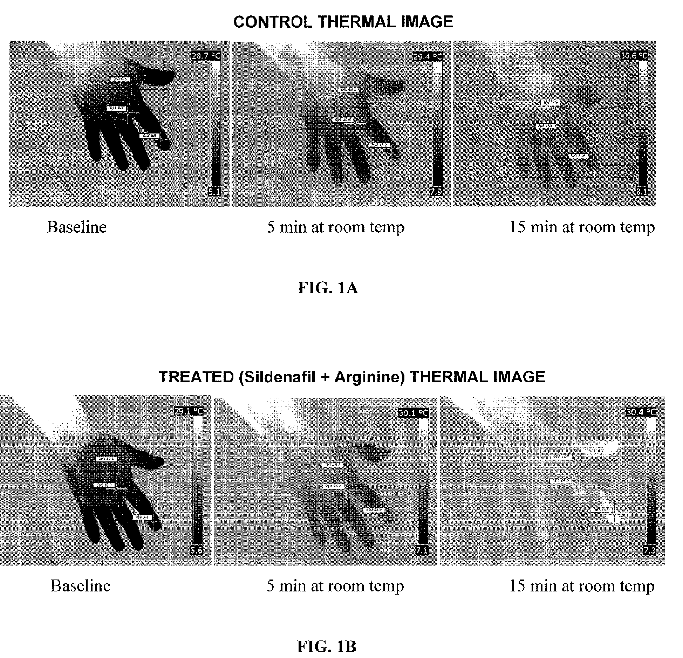

Figure 1A is a series of infrared images of the hand of a human subject at 0, 5, and 15 minutes after topical application of a control Veecogel Cream base formulation containing Krisgel 100™, Coconut oil, Capryloic Acid, Triglicerides, Squalane, Boric Acid, Polysorbate 80, Sorbitan Monooleate 80, Simethicone, Butylated hydroxytoluene.

Figure 1B is a series of infrared images of the hand of a human subject at 0, 5, and 15 minutes after topical application of a treatment formulation in Veecogel base formulation containing a combination of L-arginine Monohydrochloride 10% w/w and Sildenafil Citrate 1% w/w cream.

**DETAILED DESCRIPTION**

The invention features compositions as defined in the claims for use in methods for treating peripheral vascular diseases and related conditions, including Raynaud's phenomenon or syndrome. In one embodiment, the method involves topically administering a composition comprising at least one cyclic nucleotide phosphodiesterase inhibitor (e.g. a cGMP PDE5 inhibitor), either by itself or in combination with one or more nitric oxide (NO) donors, and/or other optional components described herein, to a patient in need of treatment. The composition may be, for example, in the form of a gel or cream suitable for topical administration. In alternative embodiments, the methods and compositions may be adapted for oral administration of the phosphodiesterase inhibitor and/or NO donor components.

The disclosure also features a kit that includes a pharmaceutical composition comprising a PDE5 inhibitor by itself or in combination with an NO donor, directions relating to the use of the composition for treating vascular diseases, and a container.

Mammalian blood vessels typically respond to nitrovasodilators in a much more pronounced manner if the systemic vascular relaxation and dilation of tissues and veins due to the presence of incipient cyclic guanosine 3',5'-monophosphate (cGMP) is not impeded by the presence of cGMP-specific degrading enzyme of phosphodiesterase type 5 (PDE5). Thus, one approach for enhancing a patient's response to a nitrovasodilator is for a chemical inhibitor of phosphodiesterase type 5 (PDES) to be present in the treatment formulation.

In addition, the synthesis of smooth tissue relaxant cyclic guanosine 3', 5'monophosphate (cGMP) may be preceded by the presence of activated enzyme-soluble guanylate cyclase (sGC), which then causes the synthesis of guanosine monophosphate (cGMP) to take place. However, the enzyme (sGC) generally needs to be activated by the presence of an active nitric oxide radical (NO) or chemicals that are capable of releasing NO in such tissues. Thus, the presence of a NO releasing agents, such as L-arginine or other nitrovasodilators, in the formulation may be desirable. The mixture can promote synthesis of NO in areas that cannot themselves produce it, thereby facilitating systemic vascular relaxation and dilation in order to enhance blood flow and vascular circulation to the skin and dermis. The release of NO stimulates the synthesis of guanosine 3' 5'-cyclic monophosphate (cGMP) in a target cell by directly activating the soluble isoform of enzyme guanylate cyclase (sGC). NO then activates the enzyme guanylate cyclase, which results in increased levels of synthesis of cyclic guanosine monophosphate (cGMP), which escapes degradation by phosphodiesterase type 5 (PDE5) enzyme, in the presence of a guanosine monophosphate (cGMP) specific phosphodiesterase type 5 (PDE5) inhibitor, such as sildenafil citrate (VIAGRAR®), which is designated chemically as 1-[[3-(6,7-dihydro-1-methyl-7-oxo-3-propyl-1H-pyrazolo[4,3-d]pyrimidin-5-yl)-4-ethoxyphenyl]sulfonyl]-4 methylpiperazine citrate.

In one embodiment of the present disclosure the combined effect of topical application of L-arginine and/or other nitrovasodilators is used to promote the release of nitric oxide (NO) to activate the enzyme guanylate cyclase (sGC) for the increased level of synthesis of guanosine 3' 5'-cyclic monophosphate, (cGMP) in the presence of cGMP-spccific PDE5 inhibitors to enhance vascular circulation and increase blood flow to the skin. In addition to nitric oxide (NO) releasing agents, such as nitroglycerin or $C_3H_5N_3O_9$, sodium nitroprusside (sodium nitroferricyanide) or $Na_2Fe(CN)_5NO-2H_2O$, pyrimidine, $C_9H_{15}N_5O$ (also known as MINOXIDIL®), or alternatively 2,4-Diamino-6-piperidinopyrimidine 3-N-oxide, (see Ignarro et al., Pharmacol. Exp. Ther., 218, 739-749 (1981); Ignarro, Annu. Rev. Pharmacol. Toxicol., 30, 535-560 (1990); Kruszyna et al., Toxicol. Apol. Pharmacol., 91, 429-438 (1987); Wilcox et al., Chern. Res. Toxicol., 3, 71-76 (1990)), there are other NO releasing compounds suitable for use in the present invention. For example, U.S. Pat. No. 6,379,660 to Saavedra, et al., entitled "Nitric oxide-releasing 1-[(2-carboxylato)pyrrolidin-1-yl]diazen-1-ium-1,2-diolates and composition comprising same," discusses a suitable polymeric composition capable of releasing nitric oxide under physiological conditions, which includes a biopolymer, such as a peptide, polypeptide, protein, oligonucleotide or nucleic acid, to which is bound a nitric oxide-releasing functional group, and pharmaceutical compositions comprising the polymeric composition. In addition, U.S. Pat. No. 6,391,895 to Towart, et al., entitled "Nitric oxide releasing chelating agents and their therapeutic use," discusses chelating agents that may be used in the present invention, such as dipyridoxyl and aminopolycarboxylic acid based chelating agents, and their metal chelates, which can be linked directly or indirectly to at least one nitric oxide releasing moiety, or used in combination with nitric oxide or a nitric oxide-releasing moiety.

At least one phosphodiesterase type 5 inhibitor and at least one nitric oxide donor for use according to the invention, as well as the methods and compositions of the disclosure can be used to treat peripheral vascular disease. Peripheral vascular disease includes diseases arising from abnormalities of the circulatory system, such as those of functional origin (e.g., vasospasms) or of structural origin (e.g., occlusions). Examples of peripheral vascular disease include primary Raynaud's phenomenon, secondary Raynaud's phenomenon, Buerger's disease, peripheral artery disease (PAD), peripheral artery occlusive disease (PAOD, claudication), acute thrombotic occlusion, aortoiliac and lower occlusive arterial disease, arterial occlusion, arteriosclerosis, atherosclerosis, brachiocephalic and upper extremity occlusive disease, Behçet's Syndrome, carotid artery disease, vasculopathy associated with diabetes, hereditary angioedema, peripheral arterial disease (PAD), pscudoxanthoma elasticum, restenosis sclerosis, scleroderma, thrombosis, varicose veins, vasculitis, and venous and lymphatic disease. Peripheral vascular disease can be caused by any number of other disease, such as diabetes, Buerger's disease, hypertension, and Raynaud's disease, including primary and secondary Raynaud's phenomenon.

Secondary Raynaud's phenomenon is generally associated with an underlying condition. Exemplary conditions include connective tissue disorders, such as systemic sclerosis, CREST syndrome (which features calcinosis, Raynaud's phenomenon, esophageal dysmotility, sclerodactyly, and telangiectasia), systemic lupus erythematosus, rheumatoid disease (e.g., rheumatoid arthritis), Sjögren's syndrome, or polymyositis; peripheral vascular disease, such as Buerger's disease; or cancer, such as malignant cancer or after treatment with chemotherapy.

Affected areas typically include extremities supplied by the peripheral vascular system, such as the hands, feet, fingers, toes, limbs, nose, and ears. Exemplary symptoms of peripheral vascular disease include: claudication, including pain, weakness, numbness, itching, or cramping in the affected area; slow healing sores, wounds, or ulcers on the affected area; changes in color (pale, blue, or red) in the affected area; changes in temperature, such as coldness, in the affected area; diminished hair or nail growth in the affected area; dry or scaly skin; aggravated symptoms with light exertion; gangrene; intermittent paresthesia; various effects on extremities, including muscle weakness, numbness, coldness, pain, loss of pulse, cyanosis, burning sensation, ulceration, tingling, itching, and swelling; high blood pressure; brittle or thickened nails; peripheral cyanosis; and gait abnormality.

At least one phosphodiesterase type 5 inhibitor and at least one nitric oxide donor for use according to the invention, as well as the methods and compositions of the disclosure can also be used for the treatment of vasculopathy and/or other complications associated with type-1 diabetes. Such conditions include, but are not limited to, foot ulcers. It is also contemplated that the compositions herein may be used to treat dermal vascular conditions including, but are not limited to, skin aging, necrotizing fasciitis, decubitus ulcers, anal fissures, diffused cutaneous systemic sclerosis, and frostbites.

Wound healing involves the repair and reconstruction of tissue, e.g., skin, muscle, neurological tissue, bone, soft tissue, internal organs, and vascular tissue. Wound healing generally does not respond to conventional treatments that are used to treat superficial cuts. Currently, there are few specific treatments to wound healing aside from keeping the area moist and providing nutrients to the area, including angiostatic steroids, sex steroids, bromelain, vitamin B-complex, vitamins A, E and C, zinc, chondroitin sulfate, copper, ornithine alpha-ketoglutarate (OKG), arginine monomers (e.g., L-arginine monomers), carnosine, and glucosamine sulfate.

Thus, the present invention also contemplates NO donors and PDE5 inhibitors for use according to the present invention as well as the methods and compositions of the disclosure in the treatment and enhancement of wound healing. Similar to wound healing, the present invention contemplates NO donors and PDE5 inhibitors for use according to the present invention as well as the methods and compositions of the disclosure in the treatment of frostbites. Frostbite is an injury caused by exposure to cold temperatures, which does not necessarily need to be below freezing. The cold temperature causes ice crystals and clots to form and can result in poor perfusion to the face and the extremities, leading to dehydration and cell death. If the exposure continues, damage may occur to underlying blood vessels, nerves, and muscles. Therefore, the methods and compositions herein can be used to treat a variety of diseases and symptoms associated with peripheral vascular disease, including treatment of wounds or frostbite, or to treat peripheral vascular disease by diminishing one or more symptoms caused by the disease. Examples of conditions associated with peripheral vascular disease include peripheral neuropathy, autonomic neuropathy, diabetic neuropathy, and vasculitis.

*NO Donors, PDE5 Inhibitors, and Combinations Thereof*

Suitable NO donors for use in the present disclosure include compounds comprising a nitric oxide moiety, including precursors to nitric oxide. Examples of such NO donors include, but are not limited to, organic nitrates, such as mono-, di-, or tri-nitrates; organic nitrate esters, including glyceryl trinitrate (also known as nitroglycerin), isosorbide 5-mononitrate, isosorbide dinitrate, pentaerythritol tetranitrate, erythrityl tetranitrate, 1,5-pentanedinitrate, methylpropylpropanediol dinitrate, propatylnitrate, methyl 2-[4-(nitrooxymethyl)benzoyl]sulfanylbenzoate (SE 175), 1,3-(nitrooxymethyl) phenyl 2-hydroxybenzoates (as described in U.S. Patent No. 6,538,033), trolnitrate, and tenitramine; and alkyl nitrites, including amyl nitrite.

Suitable NO donors also include compounds that are converted into NO within a subject or compounds that release or donate a nitric oxide moiety. Examples of such NO donors include, but are not limited to, S-nitrosylated compounds, such as S-nitroso-N-acetyl-penicillamine (SNAP), S-nitroso-N-glutathione (SNO-GLU), or S-nitroso-N-cysteine; diazenium diolates (NONOates), including 6-(2-hydroxy-1-methyl-2-nitrosohydrazino)-N-methyl-1-hexanamine (MAHMA NONOate, NOC-9), sulfo NONOate (hydroxydiazenesulfonic acid 1-oxide, disodium salt), 3,3'-(hydroxynitrosohydrazino)bis-1-propanamine (dipropylenetriamine NONOate), 2-(N,N-diethylamino)-diazenolate 2-oxide (diethylamine NONOate, diethylammonium salt or sodium salt hydrate), or 3,3-bis(aminoethyl)-1-hydroxy-2-oxo-1-triazene (DETA NONOate); vasodilators, such as sodium nitroprusside (SNP), linsidomine, linsidomine chlorohydrate, 3-morpholinosydnonimine (SIN-I), 4-phenyl-3-furoxancarbonitrile (furoxan), molsidomine, 3-(aminopropyl)-1-hydroxy-3-isopropyl-2-oxo-1-triazene (NOC-5), 3-methylsydnone-5- nitrosimine (RE 2047), 6-piperidin-1-ylpyrimidine-2,4-diamine 3-oxide (minoxidil), (2S)-1-[(2S)-2-methyl-3-sulfanylpropanoyl]pyrrolidine-2-carboxylic acid (captopril), or nitrosylated moxisylyte derivatives, such as NMI- 678-11 and NMI-937 as described in published PCT application WO 0012075; ginseng; and zizyphi fructus.

Suitable NO donors further include, but are not limited to, amino acids, such as citrulline; and arginine or derivatives thereof, including L-arginine, N-hydroxy-L-arginine, N-$\omega$-hydroxy-homo-L-arginine, carboxylic esters of N-hydroxy-L-arginine (including, but not limited to, methyl, ethyl, propyl, isopropyl, butyl, isobutyl, t-butyl and benzoyl esters), N-$\alpha$ derivatives of N-hydroxy-L-arginine (including, but not limited to, methyl, ethyl, and benzoyl derivatives, such as N-$\alpha$-methyl-N-$\gamma$-hydroxy-L-arginine, N-$\alpha$ -benzoyl-N-benzoyl-N-$\gamma$-hydroxy-L-arginine, N-$\alpha$-benzoyl-N-$\gamma$-hydroxy-L-arginine ethyl ester), N-$\gamma$-hydroxy-agmatine, N-$\gamma$ -hydroxy-L-argininic acid, as well as nitrosated and/or nitrosylated derivatives thereof. The stable N-$\gamma$-hydroxy-L-arginine nitric oxide adduct has been characterized by Hecker et al, Proc. Natl. Acad. Sci. 1995; 92: 4671-4675.

The compositions of the disclosure also include at least one cyclic nucleotide phosphodiesterase inhibitor, such as a cGMP PDE5 inhibitor. The phosphodiesterase inhibitors used in the invention can be selective or nonselective inhibitors of the PDE5 enzyme. Nonselective inhibitors include compounds that inhibit PDE 5 and other types of phosphodiesterase enzymes, such as type 1 (PDE1), type 2 (PDE2), type 3(PDE3), type 4 (PDE4), or types 6-11; and compounds that exhibit an effect on cGMP hydrolysis, such as PDE1-PDE3 inhibitors, where PDE1-PDE3 hydrolyze both cGMP and cyclic adenosine monophosphate (cAMP). Examples of nonselective PDE inhibitors include caffeine, theophylline, IBMX (3-isobutyl-1-methylxanthine), ibudilast, papaverine, luteolin, and dipyridamole.

The phosphodiesterase inhibitors used in the disclosure preferably are selective for the PDE5 enzyme. Typically, they are selective over PDE3, more typically over PDE3 and PDE4, at therapeutic dosage ranges. Preferably, the cGMP PDE5 inhibitors of the invention have a selectivity ratio greater than 100 more preferably greater than 300, over PDE3 and more preferably over both PDE3 and PDE4. The determination of selective ratio is known in the art, which is described herein.

Suitable cGMP PDE5 inhibitors for the use according to the present disclosure include, but are not limited to, the pyrazolo[4,3-d]pyrimidin-7-ones disclosed in EP-A-0463756; the pyramlo[4,3-d]pyrimidin-7-ones disclosed in EP-A-0526004; the pyrazolo[4,3-d]pyrimidin-7-ones disclosed in published international patent application WO 93/06104; the isomeric pyrazolo[3,4-d]pyrimidin-4-ones disclosed in published international patent application WO 93/07149; the quinazolin-4-ones disclosed in published international patent application WO 93/12095; the pyrido[3,2-d] pyrimidin-4-ones disclosed in published international patent application WO 94/05661; the purin-6-ones disclosed in published international patent application WO 94/00453; the pyrazolo[4,3-d]pyrimidin-7-ones disclosed in published international patent application WO 98/49166; the pyrazolo[4,3-d]pyrimidin-7-ones disclosed in published international patent application WO 99/54333; the pyrazolo[4,3d]pyrimidin-4-ones disclosed in EP-A-0995751; the pyrazolo[4,3-d]pyrimidin-7-ones disclosed in published international patent application WO 00/24745; the pyrazolo[4,3-d]pyrimidin-4-ones disclosed in EP-A-0995750; the compounds disclosed in published international application WO 95/19978; the compounds disclosed in published international application WO 99/24433;the compounds disclosed in published international application WO 93/07124; the pyrazolo[4,3-d]pyrimidin-7-ones disclosed in published international application WO 01/27112; the pyrazolo[4,3-d]pyrimidin-7-ones disclosed in published international application WO 01/27113; the compounds disclosed in EP-A-1092718; and the compounds disclosed in EP-A-1092719.

Preferred PDE5 inhibitors for the use according to the present disclosure include the following:

5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil), also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine (see EP-A-0463756);

5-(2-ethoxy-5-morpholinoacetylphenyl)-1-methyl-3-n-propyl-1,6-dihydro-7H-pyrazolo[4,3-d]pyrimidin-7-one (see EP-A-0526004);

3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7 -one (see WO 98/49166);

3-ethyl-5-[5-(4-ethylpiperazin-1-ylsulphonyl)-2-(2-methoxyethoxy) pyridin-3-yl]-2-(pyridin-2-yl)methyl-2,6-dihydro-7H-pyrazolo[4,3-d]pyrimidin-7-one (see WO 99/54333);

(+)-3-ethyl-5-[5-(4-ethylpiperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1R)-2methoxy-1-methylethyl]oxy)pyridin-3-yl}-2-methyl-2,6-dihydro-7H-pyrazolo[4,3-d]pyrimidin-7-one (see WO 99/54333);

5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see WO 01/27113, Example 8);

5-[2-iso-butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (see WO 01/27113, Example 15);

5-[2-ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see WO 01/27113, Example 66);

5-(S-acetyl-2-propoxy-3-pyridinyl)-3-ethyl-2-(1-isopropyl-3-azetidinyl)-2,6-dihydro-7H-pyrazolo[4,3-d]pyrimidin-7-one (see WO 01/27112, Example 124);

5-(5-acetyl-2-butoxy-3-pyridinyl)-3-ethyl-2-(1-ethyl-3-azetidinyl)-2,6-dihydro-7H-pyrazolo[4,3-d]pyrimidin-7-one (see WO 01/127112, Example 132);

(6R,12aR)-2,3,6,7,12,12a-hexahydro-2-methyl-6-(3,4-methylenedioxyphenyl)-pyrazino[2',1':6,1]pyrido[3,4-b]indole-1,4-dione (IC-351 or tadalafil), i.e. the compound of examples 78 and 95 of published international application WO 95/19978, as well as the compound of examples 1,3, 7 and 8;

2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil), also known as 1-[[3-(3,4-dihydro-S-methyl-4-oxo-7-propylimidazo[5,1-f]as-triazin-2-yl)-4-ethoxyphenyl]sulphonyl]-4-ethylpiperazine, i.e. the compound of examples 20, 19, 337 and 336 of published international application WO 99/24433;

the compound of example 11 of published international application WO 93/07124 (EISAI); and

compounds 3 and 14 from Rotella DP et al., J. Med. Chem. 2000; 43(7): 1257-1263.

Other cGMP PDE5 inhibitors include lodenafil, gisadenafil, avanafil, gisadenafil, mirodenafil, parogrelil, SLx-2101, udenafil, and 3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one, or derivatives or salts thereof.

Still other cGMP PDE5 inhibitors useful in conjunction with the present disclosure include, but are not limited to: 4-bromo-5-(pyridylmethylamino)-6-[3-(4-chlorophenyl)-propoxy]-3(2H)pyridazinone; 1-[4-[(1,3-benzodioxol-5-ylmethyl)amino]-6-chloro-2-quinozolinyl]-4-piperidine-carboxylic acid, monosodium salt; (+)-cis-S,6a,7,9,9,9a-hexahydro-2-[4-(trifluoromethyl)-phenylmethyl-5-methyl-cyclopent-4,5]imidazo[2,1-b]purin-4(3H)one; furaziocillin; cis-2-hexyl-5-methyl-3,4,5,6a,7,8,9,9a -octahydrocyclopent[4,5]-imidazo[2,1-b]purin-4-one; 3-acetyl-1-(2-chlorobenzyl)-2-propylindole-6-carboxylate; 3-acetyl-1-(2-chlorobenzyl)-2-propylindole-6-carboxylate; 4-bromo-S-(3-pyridylmethylamino)-6-(3-(4-chlorophenyl)propoxy)-3-(2H)pyridazinone; 1-methyl-5(5-morpholinoacetyl-2-n-propoxyphenyl)-3-n-propyl-1,6-dihydro-7H-pyrazolo(4,3-d)pyrimidin-7-one; 1-[4-[(1,3-benzodioxol-5-ylmethyl)amino]-6-chloro-2-quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. S069 (Schering Plough); GF-196960 (Glaxo Wellcome); E-8010 and E-4010 (Eisai); Bay-38-3045 & 38-9456 (Bayer); and Sch-51866.

The cGMP PDE5 inhibitor and NO donor may also be used in combination with a cyclodextrin. Cyclodextrins are known to form inclusion and non-inclusion complexes with drug molecules. Formation of a drug-cyclodextrin complex may modify the solubility, dissolution rate, and bioavailability and/or stability property of a drug molecule. Drug-cyclodextrin complexes are generally useful for most dosage forms and administration routes. As an alternative to direct complexation with the drug, the cyclodextrin may be used as an auxiliary additive, e.g. as a carrier, diluent or solubilizer. Alpha-, beta-, and gamma-cyclodextrins are most commonly used and suitable examples are described in WO-A-91/11172, WO-A-94/02518, and WO-A-98/55148.

The cGMP PDE5 inhibitor and NO donor of the invention can also be administered in combination with one or more of the following:

5-HT$_{2B}$ antagonists (e.g., sarpogrelate);

$\alpha$-adrenergic receptor antagonists, also known as alpha-adrenoceptors or $\alpha$-blockers (e.g., phenoxybenzamine, phentolamine, prazosin, and doxazosin);

β-adrenergic receptor antagonists, also known as beta-adrenoceptors or β-blockers (e.g., propranolol, metoprolol, pindolol, labetalol, and carvedilol);

Angiotensin receptor antagonists (e.g., valsartan, losartan, olmesartan, and irbcsartan);

Angiotensin converting enzyme inhibitors (e.g., captopril, enalapril, lisinopril, and ramipril);

Anticoagulants (e.g., heparin, coumadin, enoxaparin, warfarin, apixaban, and rivaroxaban);

Antidepressants (e.g., fluvoxamine, paroxetine, sertraline, desvenlafaxine, duloxetine, milnacipran, venlafaxine, bupropion, atomoxetine);

Antidiabetic agents (e.g., insulin, metformin, glipizide, glyburide, glimepiride, gliclazide, repaglinide, nateglinide, rosiglitazone, pioglitazone, miglitol, acarbose, liraglutide, vildagliptin, and sitagliptin);

Antithrombotics (e.g., aspirin, dipyridamole, clopidogrel, prasugel, and cangrelor);

Calcium channel blockers (e.g., nifedipine, amlodipine, felodipine, and diltiazem);

Cholesterol-lowering drugs (e.g., atorvastatin, pravastatin, simvastatin, and rusovastatin)

Non-steroidal anti-inflammatory agents (e.g., aspirin, ibuprofen, ketoprofen, diclofenac, naproxen, and licofelone);

Prostaglandins (e.g., epoprostenol and alprostadil);

Renin antagonist (e.g., aliskiren and antidepressants with renin antagonistic activity, such as citalopram, escitalopram, and fluoxetine);

Steroidal anti-inflammatory agents (e.g., hydrocortisone, cortisone acetate, fludrocortisone acetate, deoxycorticosterone acetate, prednisone, prednisolone, methylprednisolone, dexamethasone, betamethasone, triamcinolone, beclometasone, and aldosterone);

Thromboxane A2 agonists (e.g., ramatroban and seratrodast); and

Miscellaneous drugs, such as pentoxifylline and cilostazol.

The PDE5 inhibitors or NO donors may be optionally administered as a pharmaceutically acceptable salt, such as a non-toxic acid addition salts or metal complexes that are commonly used in the pharmaceutical industry. Examples of acid addition salts include organic acids, such as acetic, lactic, pamoic, maleic, citric, malic, ascorbic, succinic, benzoic, palmitic, suberic, salicylic, tartaric, methanesulfonic, toluenesulfonic, or trifluoroacetic acids; polymeric acids, such as tannic acid, carboxymethyl cellulose, or the like; and inorganic acid, such as hydrochloric acid, hydrobromic acid, sulfuric acid phosphoric acid, or the like. Metal complexes include zinc, iron, and the like.

The PDE5 inhibitors or NO donors may be derivatives of any amino acid described herein. Derivatives of amino acids and peptides are well known in the art. A derivative of a peptide includes a peptide containing one or more conservative substitutions selected from the following groups: Ser, Thr, and Cys; Leu, Ile, and Val; Glu and Asp; Lys and Arg; Phe and Tyr; and Gln and Asn. Conservative substitutions may also be determined by other methods, such as by the BLAST (Basic Local Alignment Search Tool) algorithm, the BLOSUM Substitution Scoring Matrix, or the BLOSUM 62 matrix.

Derivatives of amino acids and peptides also include chemically modified peptides. The amino acids or peptides, or conservative derivatives thereof, may be derivatized to contain N-terminal modifications, C-terminal modifications, internal modifications, or non-standard residues. Exemplary N-terminal, C-terminal, and internal modifications include use of a solubilizing group, such as a polyethylene glycol group; an NO moiety, such as a nitro group or a nitrosyl group; a hydrophobic group, such as a C1-C8 alkyl group, or an unsubstituted or substituted aryl group; a lipid group, such as a phosphoglyceride, a sterol, or a sphingosine; a hydrophilic group, such as a hydroxyl group or an amino group; a transmembrane signal sequence or a portion thereof; or a protein transduction domain or a portion thereof. Examples of derivatives of arginine include N-hydroxy-L-arginine, dimethylarginine, N-alkyl-L-arginine, and nitrosated and nitrosylated derivatives thereof.

The PDE5 inhibitors or NO donors may also be derivatives of any compound described herein. Derivatives of compounds are well known in the art. Derivatives of compounds include modifications within the backbone of the molecule and modifications to the pendant groups of the molecule. Modifications within the backbone of the molecule include use of substitutions selected from the following groups: O, N, and S; or C-C, C=C, and C≡C. Modifications to the pendant groups include use of substitutions selected from the following groups: H and alkyl; hydroxyl and sulfhydryl; pyridyl, pyranyl, and thiopyranyl; piperidyl, tetrahydropyranyl, and thianyl; piperazinyl, morpholinyl, dithianyl, and dioxanyl; or nitro and nitrosyl.

In certain implementations of the invention, the compounds used in the composition have appropriate properties for topical administration. For example, suitable compounds typically include those that will act locally and upon absorption will be diluted into the large blood volume of the vascular space; or produce no adverse events. Other suitable compounds include those that can provide nutrients upon absorption, such as angiostatic steroids, sex steroids, bromelain, vitamin B-complex, vitamins A, E and C, zinc, chondroitin sulfate, copper, ornithine alpha-ketoglutarate (OKG), arginine monomers, carnosine, and glucosamine sulfate.

*Dosage, Formulation, and Administration*

The compositions may conveniently be administered in unit dosage form and may be prepared by any of the methods well-known in the pharmaceutical art, for example, as described in "Remington: The Science and Practice of Pharmacy" (20th ed., ed. A. R. Gennaro, 2000, Lippincott Williams & Wilkins). The concentration of the PDE5 inhibitor and/or the NO donor in the formulation will vary depending upon a number of factors, including the dosage of the drug to be administered, and the route of administration.

The composition can be prepared by any useful method. The compositions herein may be formulated into pharmaceutically acceptable salts and/or complexes thereof. Pharmaceutically acceptable salts are non-toxic salts at the concentration at which they are administered. The preparation of such salts can facilitate the pharmacological use by altering the physical-chemical characteristics of the composition without preventing the composition from exerting its physiological effect. Examples of useful alterations in physical properties include lowering the melting point to facilitate transmucosal/transdermal administration and increasing the solubility to facilitate the administration of higher concentrations of the compound.

Pharmaceutically acceptable salts include acid addition salts, such as those containing sulfate, hydrochloride, phosphate, sulfonate, sulfamate, sulfate, acetate, citrate, lactate, tartrate, methanesulfonate, ethane sulfonate, benzenesulfonate, p-toluenesulfonate, cyclohexylsulfonate, cyclohexylsulfamate, and quinate. Pharmaceutically acceptable salts can be obtained from acids, such as hydrochloric acid, sulfuric acid, phosphoric acid, sulfonic acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfonic acid, cyclohexylsulfamic acid, and quinic acid. Such salts may be prepared by, for example, reacting the free acid or base forms of the compound with one or more equivalents of the appropriate base or acid in a solvent or medium in which the salt is insoluble. In another example, a solvent, such as water, is used to prepare the salt and the solvent is then removed in vacuo, by freeze-drying, or by exchanging the ions of an existing salt for another ion on a suitable ion exchange resin.

Pharmaceutical compositions can be formulated from an active compound and/or salts and/or combinations thereof by standard techniques using one or more suitable carriers, excipients, and diluents. See, e.g., Remington's Pharmaceutical Sciences (19th Ed. Williams & Wilkins, 1995) (incorporated herein by reference for all purposes).

The composition can be formulated in any suitable carrier. Examples of suitable carriers, excipients, and diluents include lactose, dextrose, sucrose, sorbitol, mannitol, starches, gum acacia, calcium phosphate, alginates, calcium silicate, microcrystalline cellulose, polyvinyl pyrrolidine, cellulose, tragacanth, gelatin syrup, methylcellulose, methyl and propyl hydroxybenzoates, talc, magnesium stearate, water, and mineral oil. Other additives optionally include lubricating agents, wetting agents, emulsifying and suspending agents, or preservatives. The amount of these preservatives employed is generally about 0.001 to 0.1 % by weight.

The suitability of any particular PDE5 inhibitor or NOS donor can be readily determined by evaluation of its potency and selectivity using literature methods followed evaluation of its toxicity, absorption, metabolism, pharmacokinetics, etc., in accordance with standard pharmaceutical practice.

Selectivity ratios may readily be determined by a skilled person in the arts. IC50 values for the PDE3 and PDE4 enzyme may be determined using established literature methodology, see Ballard SA et al, J. Urol. 1998; 159: 2164-2171. IC50 values for the cGMP PDE5 inhibitors may be determined using established literature methodology, for example as described in EP0463756-B1 and EP0526004-A1. Preferably, the cGMP PDES inhibitors have an IC50 for PDE5 at less than 100 nanomolar, more preferably, at less than 50 nanomolar, more preferably still at less than 10 nanomolar.

Optimization of the appropriate dosages can readily be made by one skilled in the art in light of pharmacokinetics of the compound or combination of compounds. Factors to be considered by one skilled in the art include the compound's specific activity; the severity of the condition or symptoms of the subject; the age, condition, body weight, sex, and diet of the subject; the use (or not) of concomitant therapies; and other clinical factors.

The dosage of cGMP PDE5 inhibitor in such formulations will depend on its potency, but can be expected to be in the range of from 1 to 500 mg for administration up to three times a day (e.g., 1, 5, 10, 25, 50, 100, 250, and 500 mg). For oral and parenteral administration to human patients, the daily dosage level of the cGMP PDE5 inhibitor will usually be from 5 to 500 mg (as a single dose or as a dose divided throughout the day). In the case of sildenafil, a preferred dose is in the range 10 to 100 mg (e.g. 10, 25, 50 and 100 mg), which can be administered once, twice, or three times a day (preferably once). However, the precise dose will be as determined by the prescribing physician and will depend on the age and weight of the patient and severity of the symptoms.

In one non-limiting embodiment, the composition comprises between 1% to 30% (w/w) of one or more compounds (e.g., 1%-2%, 2.5%-5%, 8%-12%, 10%-20%, or 20-30% (w/w)). Preferably, the composition comprises 0.5%-12.5% (w/w) of one or more phosphodiesterase type 5 inhibitors and from 0.5% to 12.5% of one or more nitric oxide donors. Administration may be one or multiple times daily, weekly (or at some other multiple day interval) or on an intermittent schedule, with that cycle repeated a given number of times (e.g., 2-10 cycles) or indefinitely.

The compounds of the invention may be administered, for example, by oral, parenteral, buccal, sublingual, nasal, rectal, patch, pump, or transdermal administration and the pharmaceutical compositions formulated accordingly. Parenteral administration includes intravenous, intraperitoneal, subcutaneous, intramuscular, transepithelial, nasal, intrapulmonary, intrathecal, rectal, and topical modes of administration. Parenteral administration may be by continuous infusion over a selected period of time.

In a preferred implementation, the therapeutic compounds and compositions are administered locally over the skin of affected areas. Dermal bioavailability refers to the proportion of a topically administered drug that reaches the local circulation. The factors that determine dermal bioavailability of a drug are dissolution, membrane permeability and metabolic stability. Typically, screening cascade of firstly in vitro and then in vivo techniques is used to determine oral bioavailability. Dissolution or solubilization of the drug by the aqueous contents can be predicted from in vitro solubility experiments conducted at appropriate pH. Preferably the compounds of the invention have a minimum solubility of 50 mcg/ml. Solubility can be determined by standard procedures known in the art such as described in Lipinski CA et al., Adv. Drug Deliv. Rev. 1997, 23: 3-25 (republished as Adv. Drug Deliv. Rev. 2001; 46: 3-26).

Membrane permeability refers to the passage of the compound through the cells of the skin. Lipophilicity is key property in predicting this and is defined by in vitro LogPD measurements using organic solvents and buffer. Preferably the compounds of the invention have Log D of -2 to +4, more preferably -1 to +2. The log D can be determined by standard procedures known in the art such as described in Stopher D et al., J. Pharm. Pharmacol. 1990; 42: 144.

Cell monolayer assays such as caco-2 add substantially to prediction of favorable membrane permeability in the presence of efflux transporters such as p-glycoprotein, so-called caco-2 flux. Preferably, compounds of the invention have a caco-2 flux of greater than $2x10^{-6}$ cms$^{-1}$, more preferably greater than $5x10^{-6}$ cms$^{-1}$. The caco flux value can be determined standard procedures known in the art such as described in Artursson P et al., J. Pharm. Sci. 1990; 79; 595-600.

Due to the interplay of the above processes, further support that a compound will be dermally bioavailable in humans can be gained by in vivo experiments in animals. Absolute bioavailability is determined in these studies by administering the compound separately or in mixtures on rabbit ear to determine changes of blood flow. For absolute determinations (absorbed compound), the intravenous route is also employed. The details of assessing dermal bioavailability in animals are well known in the art, where examples can be found in Guy RH et al., Pharm. Res. 1986; 3: 253-262 and Herkenne C et al., Pharm. Res. 2008; 25: 87-103.

Topical administration is a preferred route of administration for the compositions of the invention. The composition can be formulated in any pharmaceutically acceptable carrier suitable for topical application to the skin. Examples of such carriers include a solid carrier, such as alumina, clay, microcrystalline cellulose, silica, or talc; and a liquid carrier, such as an alcohol, a glycol, or a water-alcohol/glycol blend. The compounds may also be administered in liposomal formulations that allow compounds to enter the skin. Such liposomal formulations are described in U.S. Pat. Nos. 5,169,637; 5,000,958; 5,049,388; 4,975,282; 5,194,266; 5,023,087; 5,688,525; 5,874,104; 5,409,704; 5,552,155; 5,356,633; 5,032,582; 4,994,213; and PCT Publication No. WO 96/40061. Examples of other appropriate vehicles are described in U.S. Pat. No. 4,877,805 and EP Publication No. 0586106A1. Suitable vehicles of the invention may also include mineral oil, petrolatum, polydecene, stearic acid, isopropyl myristate, polyoxyl 40 stearate, stearyl alcohol, or vegetable oil.

The compositions may be provided in any useful form, such as, for example, a cream, a dusting powder, an emulsion (including a microemulsion), a foam, a gel, a hydrogel, a lotion, an ointment, a shake lotion, a shampoo, a solution, a suspension, or other typical solid, semi-solid, or liquid compositions used for application to skin. The composition may also be dermally or transdermally administered, for example, by the use of a skin patch.

Such compositions may contain other ingredients typically used in such products, such as colorants, fragrances, thickeners (e.g., xanthan gum, a fatty acid, a fatty acid salt or ester, a fatty alcohol, a modified cellulose, a modified mineral material, Krisgel 100™, or a synthetic polymer), antimicrobials, solvents, including lipophilic solvents, surfactants, detergents, gelling agents, antioxidants (e.g., $\alpha$-tocopherol), fillers, dyestuffs, viscosity-controlling agents, preservatives, humectants, emollients (e.g., natural or synthetic oils, hydrocarbon oils, waxes, or silicones), hydration agents, chelating agents, tonicity adjusting agents, demulcents, solubilizing excipients, adjuvants, dispersants, permeation enhancer agents, plasticizing agents, preservatives, stabilizers, demulsifiers, wetting agents, sunscreens, emulsifiers, moisturizers, astringents, deodorants, and optionally including anesthetics, anti-itch actives, botanical extracts, conditioning agents, darkening or lightening agents, glitter, humectants, mica, minerals, polyphenols, silicones or derivatives thereof, sunblocks, vitamins, and phytomedicinals.

The compositions can also include other like ingredients to provide additional benefits and improve the feel and/or appearance of the topical formulation. Various additives may be added to the formulations herein. Such additives include substances that serve for emulsification, preservation, wetting, improving consistency, and so forth, which are conventionally employed in pharmaceutical preparations. Other additives include compounds that have surfactant properties, either ionic or nonionic, such as sorbitan monolaurate, triethanolamine oleate, polyoxyethylenesorbitan monopalmitate, dioctyl sodium sulfosuccinate, monothioglycerol, thiosorbitol, ethylenediamine tetra acetic acid, etc. Suitable preservatives for use in the pharmaceutical preparations include benzalkonium chloride, benzethonium, phenylethyl alcohol, chlorobutanol, thimerosal, and the like.

The composition can be administered in any number of ways. For example, the composition in liquid form can be applied from absorbent pads; used to impregnate bandages and other dressings; or sprayed directly onto the affected area of the subject. In another example, the composition in solid form, including semi-solid form, can be applied from a tube; or be applied directly onto the affected area of subject. In yet another example, the composition can be contained within a single use dispenser, and the gel or ointment will be applied locally rubbed on the skin of the affected area. When the composition is in liquid form, the composition can be injected locally.

Various delivery systems can also be used to administer the composition in liquid or solid form. For example, the composition in liquid form or solid form can be applied by using an applicator to spread the composition onto the affected area. In another example, the composition may also be applied to the skin under occlusive dressing in a dermal delivery system (e.g., a transdermal patch). In yet another example, the composition is formulated as an anal suppository for treatment of fissures and hemorrhoids.

Administration of compounds in controlled release formulations may be useful where the one or more compounds have (i) a narrow therapeutic index (e.g., the difference between the plasma concentration leading to harmful side effects or toxic reactions and the plasma concentration leading to a therapeutic effect is small; (ii) a narrow slow absorption rate by or through the epithelium and/or dermis; or (iii) a short biological half-life, so that frequent dosing during a day is required in order to sustain a therapeutic level.

Many strategies can be pursued to obtain controlled release for topical formulations, in which the rate of release outweighs the rate of metabolism of the therapeutic compound. For example, controlled release can be obtained by the appropriate selection of formulation parameters and ingredients, including, e.g., appropriate controlled release compositions and coatings. Examples include oil solutions, suspensions, emulsions, microcapsules, microspheres, nanoparticles, patches, and liposomes. The pharmaceutical compositions may be formulated to provide immediate, sustained or delayed release of the compound. For applications providing slow release, certain carriers may be particularly preferred. Suitable slow release carriers may be formulated from dextrose, dextran, poly lactic acid, and various cellulose derivatives, for example ethylhydroxycellulose in the form of microcapsules.

For topical application to the skin, the PDE5 inhibitor and/or NO donor can be formulated as a suitable cream, a gel, a lotion, an ointment, a shampoo, a solution, a suspension, or a transdermal patch. Specific classes of additives commonly use in these formulations include: isopropyl myristate, sorbic acid NF powder, polyethylene glycol, phosphatidylcholine (including mixtures of phosphatidylcholine, such as phospholipon G), Krisgel 100™, distilled water, sodium hydroxide, decyl methyl sulfoxide (as a permeation enhancer agent), menthol crystals, butylated hydroxytoluene, ethyl diglycol reagent, and 95% percent (190 proof) ethanol.

In particular, compositions for topical application can further include a permeation enhancer agent, such as those described in "Percutaneous Penetration enhancers", (eds. Smith EW and Maibach HI. CRC Press 1995). Exemplary permeation enhancer agents include alkyl (N,N-disubstituted amino alkanoate) esters, such as dodecyl 2-(N,N-dimethylamino) propionate (DDAIP), which is described in patent U.S. Pat. Nos. 6,083,996 and 6,118,020, which are both incorporated herein by reference; a water-dispersible acid polymer, such as a polyacrylic acid polymer, a carbomer (e.g., Carbopol™ or Carbopol 940P™, available from B. F. Goodrich Company (Akron, Ohio)), copolymers of polyacrylic acid (e.g., Pemulen™ from B. F. Goodrich Company or Polycarbophil™ from A. H. Robbins, Richmond, Va.; a polysaccharide gum, such as agar gum, alginate, carrageenan gum, ghatti gum, karaya gum, kadaya gum, rhamsan gum, xanthan gum, and galactomannan gum (e.g., guar gum, carob gum, and locust bean gum), as well as other gums known in the art (see for instance, Industrial Gums: Polysaccharides & Their Derivatives, Whistler R. L., BeMiller J. N. (eds.), 3rd Ed. Academic Press (1992) and Davidson, R. L., Handbook of Water-Soluble Gums & Resins, McGraw-Hill, Inc., N.Y. (1980)); or combinations thereof.

Other suitable polymeric permeation enhancer agents are cellulose derivatives, such as ethyl cellulose, methyl cellulose, hydroxypropyl cellulose. Additionally, known transdermal permeation enhancer agents can also be added, if desired. Illustrative are dimethyl sulfoxide (DMSO) and dimethyl acetamide (DMA), 2-pyrrolidone, N,N-diethyl-m-toluamide (DEET), 1-dodecylazacycloheptane-2-one (Azone™, a registered trademark of Nelson Research), N,N-dimethylformamide, N-methyl-2-pyrrolidone, calcium thioglycolate and other enhancers such as dioxolanes, cyclic ketones, and their derivatives and so on.

Also illustrative are a group of biodegradable permeation enhancer agents which are alkyl N,N-2-(disubstituted amino) alkanoates as described in U.S. Pat. No. 4,980,378 and U.S. Pat. No. 5,082,866, which are both incorporated herein by reference, including tetradecyl (N,N-dimethylamino) acetate, dodecyl (N,N-dimethylamino) acetate, decyl (N,N-dimethylamino) acetate, octyl (N,N-dimethylamino) acetate, and dodecyl (N,N-diethylamino) acetate.

Particularly preferred permeation enhancer agents include isopropyl myristate; isopropyl palmitate; dimethyl sulfoxide; decyl methyl sulfoxide; dimethylalanine amide of a medium chain fatty acid; dodecyl 2-(N,N-dimethylamino) propionate or salts thereof, such as its organic (e.g., hydrochloric, hydrobromic, sulfuric, phosphoric, and nitric acid addition salts) and inorganic salts (e.g., acetic, benzoic, salicylic, glycolic, succinic, nicotinic, tartaric, maleic, malic, pamoic, methanesulfonic, cyclohexanesulfamic, picric, and lactic acid addition salts), as described in U.S. Pat. No. 6,118,020; and alkyl 2-(N,N-disubstituted amino)-alkanoates, as described in U.S. Pat. No. 4,980,378 and U.S. Pat. No.

5,082,866.

The permeation enhancer agent in this composition by weight would be in the range of 0.5% to 10 % (w/w). The most preferred range would be between 1.0% and 5% (w/w). In another embodiment, the permeation enhancer agent comprises between 0.5% -1%, 1%-2%, 2%-3%, 3%-4%, or 4%-5%, (w/w) of the composition.

For topical application to the skin, the PDE5 inhibitor and/or NO donor can be formulated as a suitable solution. Suitable solution can include one of more of an antioxidant, an antimicrobial agent, a buffer, an emulsifying agent, a lipophilic solvent, a lubricating agent, a permeation enhancer agent, a stabilizer, a suspending agent, a tonicity adjusting agent, a viscosity increasing agent, or a wetting agent.

Antioxidants that may also act as stabilizers include ascorbic acid, sodium bisulfite, potassium bisulfite, ascorbyl palmitate, butylated hydroxyanisole, butylated hydroxy toluene, potassium metabisulfite, sodium metabisulfite, sodium thiosulfate, thiourea, and the like.

Suitable buffers include boric acid, sodium and potassium bicarbonate, sodium and potassium borates, sodium and potassium carbonate, sodium acetate, sodium biphosphate, Tris, and the like, in amounts sufficient to maintain the pH between about pH 3 and about pH 9.5, most preferably between about pH 7 and pH 7.5.

Stabilizers such as chelating agents that may be used include, for example, EDTA, EGTA, DTPA, DOTA, ethylene diamine, bipyridine, 1,10-phenanthroline, crown ethers, aza crown, catechols, dimercaprol, D-penicillamine, and deferoxamine.

Suitable tonicity agents include dextran 40, dextran 70, dextrose, glycerin, potassium chloride, propylene glycol, sodium chloride, and the like, such that the sodium chloride equivalent of the solution is in the range of 9.9 +/- 0.2%.

Suitable viscosity increasing agents include dextran 40, gelatin, glycerin, hydroxyethyl cellulose, hydroxymethyl propyl cellulose, lanolin, methylcellulose, petrolatum, polyethylene glycol, polyvinyl alcohol, polyvinyl polyvinylpyrrolidone, carboxymethyl cellulose, and the like. Suitable wetting agents include polysorbate 80, polysorbate 20, poloxamer 282, and tyloxapol.

Antibacterial, antiviral, antifungal and anti-tumor agents may also be used in the pharmaceutical compositions herein. Such agents and their formulations are well known to those skilled in the art.

Solutions can further include preservatives. A carrier is preferably a sterile, substantially isotonic aqueous solution. Such solutions will typically maintain sterility by employing well-known preservatives. The amount of these preservatives employed is generally about 0.001 to 0.1 % by weight.

A preferred solution comprises a permeation enhancer agent, a buffer, and a lipophilic solvent, as described in U.S. Pat. No, 6,083,996. Examples of permeation enhancer agents are described herein. Examples of lipophilic solvents include mixture of one or more aliphatic C1-C8 alcohols (e.g., ethanol, n-propanol, isopropanol, glycerol, and propylene glycol) with one or more aliphatic C8 to C30 esters (e.g., ethyl acetate, butyl acetate, ethyl laurate, methyl propionate, isopropyl myristate, and isopropyl palmitate). Preferred mixtures include ethanol, isopropanol or propylene glycol with isopropyl myristate. Examples of buffers include an acid buffer system. Acid buffer systems serve to maintain or buffer the pH of compositions within a desired range. In one non-limiting embodiment, the solution comprises one or more PDE5 inhibitors, one or more NO donors, a permeation enhancer agent, a buffer, and a lipophilic solvent.

The composition can also be formulated as a suitable ointment containing the two compounds suspended or dissolved in, for example, a mixture with one or more of the following: mineral oil, liquid petrolatum, white petrolatum, propylene glycol, a polyoxyethylene polyoxypropylene compound, emulsifying wax, and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol, and water. The ointment can further include permeation enhancer agents and/or preservatives, where particularly suitable preservatives include methyl and propyl parabens.

The composition can also be formulated as a suitable cream. In one preferred embodiment, the base cream has properties of excellent absorption into the skin and further comprises a PDE5 inhibitor, L-arginine hydrochloride, and a permeation enhancer agent. The components of the base cream may be those commonly found in hand cream, such as water, mineral oil, glyceryl stearate, squalene, propylene glycol stearate, wheat germ oil, glyceryl stearate, isopropyl myristate, steryl stearate, polysorbate 60, propylene glycol, oleic acid, tocopherol acetate, collagen, sorbitan stearate, vitamin A, vitamin D, triethanolamine, methylparaben, aloe vera extract, imidazlidineyl urea, propylparaben, Omega-3, DHA, EPA, and BHA.

In one preferred embodiment, the composition is administered as a base cream with the properties of excellent absorption into the skin which contains NO donor (e.g., 0.5% to 12.5 % w/w of L-Arginine) and PDE-5 inhibitor.

Alternatively, the compositions of the invention may be adapted for other routes of administration, such as oral administration. In humans, cGMP PDE5 inhibitors are typically given orally to the patient. In circumstances where the recipient suffers from a swallowing disorder or from impairment of drug absorption after oral administration, the drug may be administered parenterally, sublingually, or buccally. In another embodiment, the compositions of the invention are adapted for delivering the PDE5 inhibitor and the NO donor using different routes of administration. For example, the PDE5 inhibitor is formulated for oral administration and the NO donor is formulated for topical formulation.

Suitable oral formulations include tablets, capsules, pills, powders, granules, dragees, gels, slurries, ointments, solutions suppositories, injections, inhalants and aerosols. The compounds of the invention can be admixed with a pharmaceutically acceptable carrier adapted for oral administration. Examples of such pharmaceutically acceptable carriers include an orally ingestible carrier, such as gelatin; inert diluents, such as calcium carbonate, sodium carbonate, lactose, calcium phosphate, sodium phosphate, or kaolin; binders; or lubricants. For soft gelatin capsules, the active ingredient is mixed with water or an oil medium.

Compositions intended for oral administration may be prepared according to any method known to the art for the manufacture of pharmaceutical compositions. The compositions may optionally contain sweetening agents, flavoring agents, coloring agents, and preserving agents in order to provide a more palatable preparation.

For oral administration, fine powders or granules may contain diluting, dispersing, and/or surface active agents. The oral formulation may be in any form, including in water or in a syrup; in capsules or sachets in the dry state; in a non-aqueous solution or suspension, wherein suspending agents may be included; in tablets, wherein binders and lubricants may be included; or in a suspension with water or with syrup. Wherever required, flavoring, preserving, suspending, thickening, or emulsifying agents may also be included. Tablets, capsules, and pills are preferred oral administration forms, and these may be coated.

By way of example, extended or modified release oral formulation can be prepared using additional methods known in the art. For example, a suitable extended release form of the either active pharmaceutical ingredient or both may be a matrix tablet or capsule composition. Suitable examples of matrix forming materials include waxes (e.g., carnauba, bees wax, paraffin wax, ceresine, shellac wax, fatty acids, and fatty alcohols), oils, hardened oils or fats (e.g., hardened rapeseed oil, castor oil, beef tallow, palm oil, and soya bean oil), and polymers (e.g., hydroxypropyl cellulose, polyvinylpyrrolidone, hydroxypropyl methyl cellulose, and polyethylene glycol). Other suitable matrix tabletting materials are microcrystalline cellulose, powdered cellulose, hydroxypropyl cellulose, ethyl cellulose, with other carriers, and fillers. Tablets may also contain granulates, coated powders, or pellets. Tablets may also be multi-layered. Multi-layered tablets are especially preferred when the active ingredients have markedly different pharmacokinetic profiles. Optionally, the finished tablet may be coated or uncoated.

The coating composition typically contains an insoluble matrix polymer (e.g., approximately 15-85% by weight of the coating composition) and a water soluble material (e.g., approximately 15-85% by weight of the coating composition). Optionally an enteric polymer (e.g., approximately 1 to 99% by weight of the coating composition) may be used or included. Suitable water soluble materials include polymers, such as polyethylene glycol, hydroxypropyl cellulose, hydroxypropyl methyl cellulose, polyvinylpyrrolidone, polyvinyl alcohol; monomeric materials, such as sugars (e.g., lactose, sucrose, fructose, mannitol, and the like); salts (e.g., sodium chloride, potassium chloride, and the like); organic acids (e.g., fumaric acid, succinic acid, lactic acid, and tartaric acid); and mixtures thereof. Suitable enteric polymers include hydroxypropyl methyl cellulose, acetate succinate, hydroxypropyl methyl cellulose, phthalate, polyvinyl acetate phthalate, cellulose acetate phthalate, cellulose acetate trimellitate, shellac, zein, and polymethacrylates containing carboxyl groups.

The coating composition may be plasticized according to the properties of the coating blend such as the glass transition temperature of the main component or mixture of components or the solvent used for applying the coating compositions. Suitable plasticizers may be added from 0 to 50% by weight of the coating composition and include, for example, diethyl phthalate, citrate esters, polyethylene glycol, glycerol, acetylated glycerides, acetylated citrate esters, dibutylsebacate, and castor oil. If desired, the coating composition may include a filler. The amount of the filler may be 1% to approximately 99% by weight based on the total weight of the coating composition and may be an insoluble material such as silicon dioxide, titanium dioxide, talc, kaolin, alumina, starch, powdered cellulose, microcrystalline cellulose, or polacrilin potassium.

The coating composition may be applied as a solution or latex in organic solvents or aqueous solvents or mixtures thereof. If solutions are applied, the solvent may be present in amounts from approximate by 25-99% by weight based on the total weight of dissolved solids. Suitable solvents are water, lower alcohol, lower chlorinated hydrocarbons, ketones, or mixtures thereof. If latexes are applied, the solvent is present in amounts from approximately 25-97% by weight based on the quantity of polymeric material in the latex. The solvent may be predominantly water.

Further features and advantages of this invention are further illustrated by the following examples, which are in no way intended to be limiting thereof.

**EXAMPLES**

Example 1: Preparation of a Topical Cream

A penetrating cream is prepared containing effective concentrations of L-arginine and a PDE5 inhibitor with a salt, such as sodium chloride, at a concentration sufficient to produce a hostile biophysical environment for L-arginine and the PDE5 inhibitor. The cream is applied to the tissue. Within 20 minutes, the cream begins to exert a warming effect that is prolonged, often lasting from about 2-18 hours.

Example 2: Effects of Topical Application of Arginine and Sildenafil in Pigs

The effects of topical application of arginine and sildenafil were tested using domesticated pig because pig skin is generally considered to be the closest to that of humans of any experimental animal. 12 piglets (16-26 kg) were anesthetized using intraperitoneal thiamylal (25-40 mg/kg). A stable depth of sedation was maintained with continuous intravenous methohexital, titrated to a minimal lid reflex and a regular respiratory rate. Throughout the experiment, the animals spontaneously breathed through a "snout-cone" connected to a standard anesthesia machine delivering 100% oxygen.

The dorsal surface of the animal was cleaned with tepid water. Ten sites (2 x 2 cm each) were defined on the dorsal surface of the animal with permanent marker. Blood pressure was stabilized between 60 and 80 mmHg for at least 10 minutes prior to recording baseline Doppler images. Adjustments to anesthesia and fluid loading were used throughout the procedure to attempt to maintain mean arterial pressure (MAP) within this range (60-80 mmHg). MAP was recorded at the start and end of each Doppler scan.

A dose volume of 0.1 mL of vehicle, control, or test article was applied to each site. L-arginine monohydrate and sildenafil citrate were prepared in commercially available Veecogel Cream base formulation (available from PCCA, 9901 South Wilcrest Drive, Houston, Texas, USA) containing Krisgel 100™, Coconut oil, Capryloic Acid, Triglicerides, Squalane, Boric Acid, Polysorbate 80, Sorbitan Monooleate 80, Simethicone, Butylated hydroxytoluene. Sildenafil citrate 1% was dissolved in propylene glycol 2% and combine with base with constant mixing. L-arginine monohydrate 10% and 1% were dissolved in 2% propylene glycol and combined with the Veecogel described above.

No two adjacent sites were administered the same concentration of any control or test article and administration was temporally spaced to allow at least 2 minutes between dosing each site. Regional blood flow measurements were performed by trained research associates using the PeriScan PIM 3 Laser Doppler System (Perimed AB, Järfälla, Sweden). Applications were made in the same order and over the same time span as the baseline blood flow measurements. Blood flow was measured at all ten sites at 0, 10, 20, 30, 60, 120 and 180 minutes after application. Blood flow at the untreated, control site was measured immediately prior to and immediately after the measurements at the treated sites. These two measurements were averaged to obtain the control blood flow for each interval. Serum levels of local drugs were not measured. The results are provided in Table 1 below.

Regional differences in blood flow were accounted for by normalizing the measurements of flow made after injection of a test solution to the baseline flow at the site prior to application. Changes in blood flow are expressed as a percent change from control blood flow. Variations in skin blood flow over the course of the experiment were accounted for by normalizing the changes in blood flow at a given site to the changes in blood flow at a control site. Thus, the change in cutaneous blood flow at a treatment site can be determined by the formula:

$$\% \text{ change In blood flow} = \frac{S_x - S_0 - \dfrac{C_x - C_0}{C_0}}{S_0} \times 100$$

where $S_x$ is the blood flow at an injected site X min after application, $S_0$ is the baseline flow at that site prior to application, $C_x$ is the flow at the control site X min after the application, and $C_0$ is the blood flow at the control site at the same time that the baseline flow measurements were made.

Topical application of the nitrous oxide donor L-arginine monohydrochloride increased dermal blood flow in a dose and time dependent manner. The cGMP- phosphodiesterase inhibitor sildenafil citrate at a topical concentration of 1% in Veecogel cream base also increased blood flow in the areas of application. Under the current conditions the effect of L-arginine was greater than that seen with sildenafil, attesting to the need for GC activation for sustained physiological response. Combination of low dose L-arginine with a minimally effective dose of sildenafil produced a robust response indicting that addition of a nitrous oxide donor along with an inhibitor of cGMP hydrolysis results in a synergist physiological response.

Table 1. Blood flow at each treatment site before and after (5, 10, 20, 30, 60, 120 and 180 min.) application of drug or inactive control gel.

| LOCAL CUTANEOUS BLOOD FLOW IN PIGS | | | | | | | | |
|---|---|---|---|---|---|---|---|---|
| DRUG | DOSE | FLOW (MV) Time after treatment | | | | | | |
| | | Baseline | 10 min. | 20 min. | 30 min. | 60 min. | 120 min | 180 min. |
| Arginine | 1% | 0.52 | 0.41 | 12.47 | 5.55 | 2.71 | 7.91 | 10.26 |
| Arginine | 10% | 3.4 | 19.82 | 21.88 | 22.07 | 38.45 | 40.21 | 38.72 |
| Sildenafil | 1% | -13.58 | -10.31 | -3.51 | -1.57 | 1.58 | 3.15 | 15.73 |
| Combination | Arginine 1% + Sildenafil 1% | 2.24 | 10.00 | 21.79 | 46.20 | 50.32 | 51.74 | 55.73 |

Example 3: Effects of L-Arginine and Sidelnafil Cirtrate in Humans

The effects of L-arginine and sidelnafil citrate in the regulation of human blood flow was determined by measuring the rate of skin temperature recovery in the hands of normal human volunteers following a period of cold water immersion. The measurements were performed at room temperature 25°C, for a period of 30 minutes after acclimatization. L-arginine monohydrate and sildenafile citrate were prepared in commericaly available Veecogel Cream base formulation containing Krisgel 100™, Coconut oil, Capryloic Acid, Triglicerides, Squalane, Boric Acid, Polysorbate 80, Sorbitan Monooleate 80, Simethicone, Butylated hydroxytoluene. Sildenafil citrate 1% was dissolved in propylene glycol 2% and combine with base with constant mixing. L-arginine monohydrate 10% and 1% were dissolve in 2% propylene glycol and combine with the Veecogel described above.

Skin temperature was measured with an infrared camera FLIR T300 (FLIR Systems, Inc., Boston, Massachusetts, USA). Changes in skin temperature were determined after cooling of hand for 2 minutes in ice water by analyzing individual thermal images with FLIR Quick Report software. The effects of test drugs were determined following application of substance to the cooled hand. The results are reported in Table 2 below. Thermal images of a patient receiving the control treatment and of a patient receiving the combination treatment were taken at 0, 5, and 15 minutes after treatment, and are shown in FIG. 1A and 1B, respectively.

Table 2. Dorsal skin temperature of hand following immersion in ice water. Baseline readings indicate starting hand temperature after cooling for 2 minutes. Readings at 1 to 30 minutes reflect differences from baseline values in response to treatment and acclimation at 25°C after removal from cold water.

| CHANGE IN SKIN TEMPERAURE FOLLOWING COLD WATER IMMERSION AND ROOM TEMPERATURE ACCLIMATION | | | | | | | | |
|---|---|---|---|---|---|---|---|---|
| DRUG | DOSE | Baseline Temp °C | 1 min. | 2 min. | 5 min. | 10 min. | 15 min. | 30 min. |
| Control | | 7.35 | 2.4 | 3.45 | 7.4 | 9.6 | 10.45 | 11.15 |
| Arginine | 10% W/v | 12 | 2.5 | 4 | 5 | 9.5 | 11.5 | |
| Sildenafil | 1 % W/v | 14 | 1 | 4 | 6.5 | 8.5 | 12.5 | |
| Combination | 10% Arginine + 1% Sildenafil | 8 | 2.65 | 4.1 | 8.6 | 12.1 | 18.6 | |

Claims

1. At least one phosphodiesterase type 5 inhibitor and at least one nitric oxide donor for use in a method of treating a peripheral vascular disease, wherein the phosphodiesterase type 5 inhibitor is sildenafil, derivatives, or salts thereof and the nitric oxide donor is arginine, derivatives, or salts thereof, and wherein the sildenafil, derivatives, or salts thereof and the arginine, derivatives, or salts thereof are administered in combination to a subject to treat the peripheral vascular disease.

2. The at least one phosphodiesterase type 5 inhibitor and at least one nitric oxide donor for use according to claim 1, wherein the sildenafil, derivatives, or salts thereof and the arginine, derivatives, or salts thereof are administered topically or orally.

3. The at least one phosphodiesterase type 5 inhibitor and at least one nitric oxide donor for use according to claim 1, wherein the sildenafil is sildenafil citrate.

4. The at least one phosphodiesterase type 5 inhibitor and at least one nitric oxide donor for use according to claim 1, wherein the sildenafil, derivatives, or salts thereof has an IC50 of less than 100 nanomolar; or wherein the sildenafil, derivatives, or salts thereof has a selectivity ratio in excess of 1000.

5. The at least one phosphodiesterase type 5 inhibitor and at least one nitric oxide donor for use according to claim 1, wherein the arginine is L-arginine or wherein the salt of arginine is monohydrochloride.

6. The at least one phosphodiesterase type 5 inhibitor and at least one nitric oxide donor for use according to claim 1, further comprising administering one or more of a 5-HT$_{2B}$ antagonist, an $\alpha$-adrenergic receptor antagonist, a $\beta$-adrenergic receptor antagonist, an angiotensin receptor antagonist, an angiotensin converting enzyme inhibitor, an anticoagulant, an antidepressant, an antidiabetic agent, an antithrombotic, a calcium channel blocker, a cholesterol-lowering drug, a non-steroidal anti-inflammatory agent, a prostaglandin, a rennin antagonist, a steroidal anti inflammatory agent, or a thromboxane A2 agonist.

7. The at least one phosphodiesterase type 5 inhibitor and at least one nitric oxide donor for use according to claim 2, wherein the sildenafil, derivatives, or salts thereof and the arginine, derivatives, or salts thereof are administered orally and the sildenafil, derivatives, or salts thereof is present in an amount from 1 mg to 500 mg and the arginine, derivatives, or salts thereof is present in an amount from 1 mg to 500 mg.

8. The at least one phosphodiesterase type 5 inhibitor and at least one nitric oxide donor for use according to claim 2, wherein the sildenafil, derivatives, or salts thereof and the arginine, derivatives, or salts thereof are administered topically and the sildenafil, derivatives, or salts thereof is present in an amount from 0.5% w/w to 12.5% w/w and the arginine, derivatives, or salts thereof is present in an amount from 0.5% w/w to 12.5% w/w.

9. The at least one phosphodiesterase type 5 inhibitor and at least one nitric oxide donor for use according to claim 1, wherein sildenafil citrate in an amount of 1% w/w and L-arginine monohydrochloride in an amount of 1% w/w are administered in combination.

10. The at least one phosphodiesterase type 5 inhibitor and at least one nitric oxide donor for use according to claim 1, wherein the sildenafil, derivatives, or salts thereof and the arginine, derivatives, or salts thereof when administered in combination results in a synergistic increase in cutaneous blood flow thereby treating the peripheral vascular disease.

11. The at least one phosphodiesterase type 5 inhibitor and at least one nitric oxide donor for use according to claim 1, wherein the peripheral vascular disease is primary or secondary Raynaud's phenomenon.

**Patentansprüche**

1. Mindestens ein Phosphodiesterase-Typ-5-Inhibitor und mindestens ein Stickstoffoxiddonor zur Verwendung in einem Verfahren zur Behandlung einer peripheren vaskulären Erkrankung, wobei der Phosphodiesterase-Typ-5-Inhibitor Sildenafil, Derivate oder Salze davon ist und der Stickstoffoxiddonor Arginin, Derivate oder Salze davon ist und wobei das Sildenafil, die Derivate oder Salze davon und das Arginin, die Derivate oder Salze davon in Kombination an ein Subjekt verabreicht werden, um die periphere vaskuläre Erkrankung zu behandeln.

2. Mindestens ein Phosphodiesterase-Typ-5-Inhibitor und mindestens ein Stickstoffoxiddonor zur Verwendung gemäß Anspruch 1, wobei das Sildenafil, die Derivate oder Salze davon und das Arginin, die Derivate oder Salze davon topisch oder oral verabreicht werden.

3. Mindestens ein Phosphodiesterase-Typ-5-Inhibitor und mindestens ein Stickstoffoxiddonor zur Verwendung gemäß Anspruch 1, wobei das Sildenafil Sildenafilcitrat ist.

4. Mindestens ein Phosphodiesterase-Typ-5-Inhibitor und mindestens ein Stickstoffoxiddonor zur Verwendung gemäß Anspruch 1, wobei das Sildenafil, die Derivate oder Salze davon einen IC50 von weniger als 100 nanomolar haben oder wobei das Sildenafil, die Derivate oder Salze davon ein Selektivitätsverhältnis von über 1000 haben.

5. Mindestens ein Phosphodiesterase-Typ-5-Inhibitor und mindestens ein Stickstoffoxiddonor zur Verwendung gemäß Anspruch 1, wobei das Arginin L-Arginin ist oder wobei das Salz von Arginin Monohydrochlorid ist.

6. Mindestens ein Phosphodiesterase-Typ-5-Inhibitor und mindestens ein Stickstoffoxiddonor zur Verwendung gemäß Anspruch 1, weiterhin umfassend die Verabreichung von einem oder mehreren eines 5-HT$_{2B}$-Antagonisten, eines $\alpha$-adrenergen Rezeptorantagonisten, eines $\beta$-adrenergen Rezeptorantagonisten, eines Angiotensinrezeptor-Antagonisten, eines Angiotensinkonversionsenzym ("angiotensin converting enzyme")-Inhibitors, eines Antikoagulans, eines Antidepressivums, eines antidiabetischen Mittels, eines antithrombotischen Mittels, eines Kalziumkanalblockers, eines cholesterinsenkenden Wirkstoffs, eines nichtsteroidalen anti-inflammatorischen Mittels, eines Prostaglandins, eines Renninantagonisten, eines steroidalen anti-inflammatorischen Mittels oder eines Thromboxan-A2-Agonisten.

7. Mindestens ein Phosphodiesterase-Typ-5-Inhibitor und mindestens ein Stickstoffoxiddonor zur Verwendung gemäß Anspruch 2, wobei das Sildenafil, die Derivate oder Salze davon und das Arginin, die Derivate oder Salze davon oral verabreicht werden und das Sildenafil, die Derivate oder Salze davon in einer Menge von 1 mg bis 500 mg vorhanden ist und das Arginin, die Derivate oder Salze davon in einer Menge von 1 mg bis 500 mg vorhanden ist.

8. Mindestens ein Phosphodiesterase-Typ-5-Inhibitor und mindestens ein Stickstoffoxiddonor zur Verwendung gemäß Anspruch 2, wobei das Sildenafil, die Derivate oder Salze davon und das Arginin, die Derivate oder Salze davon topisch verabreicht werden und das Sildenafil, die Derivate oder Salze davon in einer Menge von 0,5 Gew.-% (Gew./Gew.) bis 12,5 Gew.-% (Gew./Gew.) vorhanden ist und das Arginin, die Derivate oder Salze davon in einer Menge von 0,5 Gew.-% (Gew./Gew.) bis 12,5 Gew.-% (Gew./Gew.) vorhanden ist.

9. Mindestens ein Phosphodiesterase-Typ-5-Inhibitor und mindestens ein Stickstoffoxiddonor zur Verwendung gemäß Anspruch 1, wobei das Sildenafilcitrat in einer Menge von 1 Gew.-% (Gew./Gew.) und L-Argininmonohydrochlorid in einer Menge von 1 Gew.-% (Gew./Gew.) in Kombination verabreicht werden.

10. Mindestens ein Phosphodiesterase-Typ-5-Inhibitor und mindestens ein Stickstoffoxiddonor zur Verwendung gemäß Anspruch 1, wobei das Sildenafil, die Derivate oder Salze davon und das Arginin, die Derivate oder Salze davon, wenn sie in Kombination verabreicht werden, zu einem synergistischen Anstieg des kutanen Blutflusses führen, wodurch die periphere vaskuläre Erkrankung behandelt wird.

11. Mindestens ein Phosphodiesterase-Typ-5-Inhibitor und mindestens ein Stickstoffoxiddonor zur Verwendung gemäß Anspruch 1, wobei die periphere vaskuläre Erkrankung primäres oder sekundäres Raynaud-Syndrom ist.

**Revendications**

1. Au moins un inhibiteur de phosphodiestérase de type 5 et au moins un donneur d'oxyde nitrique pour leur utilisation dans un procédé de traitement d'une maladie vasculaire périphérique, l'inhibiteur de phosphodiestérase de type 5 étant le sildenafil, des dérivés ou sels de celui-ci et le donneur d'oxyde nitrique étant l'arginine, des dérivés ou sels de celle-ci , et le sildenafil, des dérivés ou sels de celui-ci et l'arginine, des dérivés ou sels de celle-ci étant administrés en association à un sujet pour traiter la maladie vasculaire périphérique.

2. Au moins un inhibiteur de phosphodiestérase de type 5 et au moins un donneur d'oxyde nitrique pour leur utilisation selon la revendication 1, le sildenafil, des dérivés ou sels de celui-ci et l'arginine, des dérivés ou sels de celle-ci étant administrés par voie topique ou orale.

3. Au moins un inhibiteur de phosphodiestérase de type 5 et au moins un donneur d'oxyde nitrique pour leur utilisation selon la revendication 1, le sildenafil étant du citrate de sildenafil.

**4.** Au moins un inhibiteur de phosphodiestérase de type 5 et au moins un donneur d'oxyde nitrique pour leur utilisation selon la revendication 1, le sildenafil, des dérivés ou sels de celui-ci ayant une CI50 inférieure à 100 nanomolaire ; ou le sildenafil, des dérivés ou sels de celui-ci ayant un rapport de sélectivité supérieur à 1 000.

**5.** Au moins un inhibiteur de phosphodiestérase de type 5 et au moins un donneur d'oxyde nitrique pour leur utilisation selon la revendication 1, l'arginine étant la L-arginine ou le sel d'arginine étant un monohydrochlorure.

**6.** Au moins un inhibiteur de phosphodiestérase de type 5 et au moins un donneur d'oxyde nitrique pour leur utilisation selon la revendication 1, comprenant en outre l'administration d'un ou plusieurs antagoniste de 5-HT$_{2B}$, un antagoniste de récepteur $\alpha$-adrénergique, un antagoniste de récepteur $\beta$-adrénergique, un antagoniste de récepteur d'angiotensine, un inhibiteur de l'enzyme de conversion de l'angiotensine, un anticoagulant, un antidépresseur, un agent antidiabétique, un antithrombotique, un bloqueur des canaux calciques, un médicament abaisseur de cholestérol, un agent anti-inflammatoire non stéroïdien, une prostaglandine, un antagoniste de la rénine, un agent anti-inflammatoire stéroïdien, ou un agoniste du thromboxane A$_2$.

**7.** Au moins un inhibiteur de phosphodiestérase de type 5 et au moins un donneur d'oxyde nitrique pour leur utilisation selon la revendication 2, le sildenafil, des dérivés ou sels de celui-ci et l'arginine, des dérivés ou sels de celle-ci étant administrés par voie orale et le sildenafil, des dérivés ou sels de celui-ci étant présents en une quantité de 1 à 500 mg et l'arginine, des dérivés ou sels de celle-ci étant présents en une quantité de 1 à 500 mg.

**8.** Au moins un inhibiteur de phosphodiestérase de type 5 et au moins un donneur d'oxyde nitrique pour leur utilisation selon la revendication 2, le sildenafil, des dérivés ou sels de celui-ci et l'arginine, des dérivés ou sels de celle-ci étant administrés par voie topique et le sildenafil, des dérivés ou sels de celui-ci étant présents en une quantité de 0,5 % poids/poids à 12,5 % poids/poids et l'arginine, des dérivés ou sels de celle-ci étant présents en une quantité de 0,5 % poids/poids à 12,5 % poids/poids.

**9.** Au moins un inhibiteur de phosphodiestérase de type 5 et au moins un donneur d'oxyde nitrique pour leur utilisation selon la revendication 1, le citrate de sildenafil en une quantité de 1 % poids/poids et le monohydrochlorure de L-arginine en une quantité de 1 % poids/poids étant administrés en association.

**10.** Au moins un inhibiteur de phosphodiestérase de type 5 et au moins un donneur d'oxyde nitrique pour leur utilisation selon la revendication 1, le sildenafil, des dérivés ou sels de celui-ci et l'arginine, des dérivés ou sels de celle-ci, administrés en association, résultant en un accroissement synergique du flux sanguin cutané traitant ainsi la maladie vasculaire périphérique.

**11.** Au moins un inhibiteur de phosphodiestérase de type 5 et au moins un donneur d'oxyde nitrique pour leur utilisation selon la revendication 1, la maladie vasculaire périphérique étant le phénomène de Raynaud primaire ou secondaire.

## CONTROL THERMAL IMAGE

| Baseline | 5 min at room temp | 15 min at room temp |

## FIG. 1A

## TREATED (Sildenafil + Arginine) THERMAL IMAGE

| Baseline | 5 min at room temp | 15 min at room temp |

## FIG. 1B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6379660 B, Saavedra
- US 6391895 B, Towart
- US 6538033 B
- WO 0012075 A
- EP 0463756 A
- EP 0526004 A
- WO 9306104 A
- WO 9307149 A
- WO 9312095 A
- WO 9405661 A
- WO 9400453 A
- WO 9849166 A
- WO 9954333 A
- EP 0995751 A
- WO 0024745 A
- EP 0995750 A
- WO 9519978 A
- WO 9924433 A
- WO 9307124 A
- WO 0127112 A
- WO 0127113 A
- EP 1092718 A
- EP 1092719 A
- WO 01127112 A
- WO 9626940 A
- WO 9111172 A
- WO 9402518 A
- WO 9855148 A
- EP 0463756 B1
- EP 0526004 A1
- US 5169637 A
- US 5000958 A
- US 5049388 A
- US 4975282 A
- US 5194266 A
- US 5023087 A
- US 5688525 A
- US 5874104 A
- US 5409704 A
- US 5552155 A
- US 5356633 A
- US 5032582 A
- US 4994213 A
- WO 9640061 A
- US 4877805 A
- EP 0586106 A1
- US 6083996 A
- US 6118020 A
- US 4980378 A
- US 5082866 A

**Non-patent literature cited in the description**

- **IGNARRO et al.** *Pharmacol. Exp. Ther.,* 1981, vol. 218, 739-749
- **IGNARRO.** *Annu. Rev. Pharmacol. Toxicol.,* 1990, vol. 30, 535-560
- **KRUSZYNA et al.** *Toxicol. Apol. Pharmacol.,* 1987, vol. 91, 429-438
- **WILCOX et al.** *Chern. Res. Toxicol.,* 1990, vol. 3, 71-76
- **HECKER et al.** *Proc. Natl. Acad. Sci.,* 1995, vol. 92, 4671-4675
- **ROTELLA DP et al.** *J. Med. Chem.,* 2000, vol. 43 (7), 1257-1263
- **REMINGTON.** The Science and Practice of Pharmacy. Lippincott Williams & Wilkins, 2000
- Remington's Pharmaceutical Sciences. Williams & Wilkins, 1995
- **BALLARD SA et al.** *J. Urol.,* 1998, vol. 159, 2164-2171
- **LIPINSKI CA et al.** *Adv. Drug Deliv. Rev.,* 1997, vol. 23, 3-25
- *Adv. Drug Deliv. Rev.,* 2001, vol. 46
- **STOPHER D et al.** *J. Pharm. Pharmacol.,* 1990, vol. 42, 144
- **ARTURSSON P et al.** *J. Pharm. Sci.,* 1990, vol. 79, 595-600
- **GUY RH et al.** *Pharm. Res.,* 1986, vol. 3, 253-262
- **HERKENNE C et al.** *Pharm. Res.,* 2008, vol. 25, 87-103
- Percutaneous Penetration enhancers. CRC Press, 1995
- Industrial Gums: Polysaccharides & Their Derivatives. Academic Press, 1992
- **DAVIDSON, R. L.** Handbook of Water-Soluble Gums & Resins. McGraw-Hill, Inc, 1980